(19) Europäisches Patentamt
European Patent Office
Office européen des brevets

(11) EP 3 543 347 B1

(12) # EUROPEAN PATENT SPECIFICATION

(45) Date of publication and mention of the grant of the patent:
**06.09.2023 Bulletin 2023/36**

(21) Application number: **18305325.5**

(22) Date of filing: **22.03.2018**

(51) International Patent Classification (IPC):
***C12Q 1/00*** *(2006.01)*

(52) Cooperative Patent Classification (CPC):
**C12Q 1/005**

(54) **METHOD OF ELECTRO-CROSS-LINKING A PROTEIN WITH A POLYPHENOL FOR THE MANUFACTURE OF A BIOSENSOR**

VERFAHREN ZUM ELEKTROVERNETZEN EINES PROTEINS MIT EINER POLYPHENOL FÜR DIE HERSTELLUNG EINER BIOSENSOR

PROCÉDÉ POUR LA ELECTRO-RÉTICULATION D'UNE PROTÉINE AVEC UN POLYPHENOL POUR LA PRODUCTION D'UN BIOCAPTEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**25.09.2019 Bulletin 2019/39**

(73) Proprietors:
- **Université de Strasbourg**
  **67000 Strasbourg (FR)**
- **CENTRE NATIONAL DE LA RECHERCHE SCIENTIFIQUE -CNRS-**
  **75016 Paris (FR)**

(72) Inventors:
- **Boulmedais, Fouzia**
  **67100 Strasbourg (FR)**
- **Schaaf, Pierre**
  **67120 Molsheim (FR)**
- **Jierry, Loïc**
  **67100 Strasbourg (FR)**
- **El Maiss, Janwa**
  **45000 Orleans (FR)**
- **Lupattelli, Paolo**
  **00144 Roma (IT)**
- **Cuccarese, Marco**
  **85100 Potenza (IT)**

(74) Representative: **Lavoix**
**2, place d'Estienne d'Orves**
**75441 Paris Cedex 09 (FR)**

(56) References cited:
**US-A1- 2003 104 119**

- **SIEBERT K J: "Effects of portein-polyphenol interactions on beverage haze, stabilisation, and analysis", JOURNAL OF AGRICULTURAL AND FOOD CHEMISTRY, AMERICAN CHEMICAL SOCIETY, BOOKS AND JOURNALS DIVISION, US, vol. 47, no. 2, 1 February 1999 (1999-02-01), pages 353-362, XP002454457, ISSN: 0021-8561, DOI: 10.1021/JF980703O**
- **EDDY S ET AL: "THE MODIFICATION OF ENZYME ELECTRODE PROPERTIES WITH NON-CONDUCTINGELECTROPOLYMERISED FILMS", BIOSENSORS AND BIOELECTRONICS, ELSEVIER BV, NL, vol. 10, no. 9/10, PT01/02, 4 December 1994 (1994-12-04), pages 831-839, XP000964972, ISSN: 0956-5663, DOI: 10.1016/0956-5663(95)99222-7**
- **BARTLETT P N ET AL: "Strategies for the development of amperometric enzyme electrodes", BIOSENSORS, ELSEVIER, BARKING, GB, vol. 3, no. 6, 1 January 1987 (1987-01-01) , pages 359-379, XP026593616, ISSN: 0265-928X, DOI: 10.1016/0265-928X(87)80018-4 [retrieved on 1987-01-01]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **BARLETT P N ET AL: "A review of the immobilization of enzymes in electropolymerized films", JOURNAL OF ELECTROANALYTICAL CHEMISTRY AND INTERFACIAL ELECTROCHEMISTRY, ELSEVIER, AMSTERDAM, NL, vol. 362, no. 1-2, 30 December 1993 (1993-12-30), pages 1-12, XP026599596, ISSN: 0022-0728, DOI: 10.1016/0022-0728(93)80001-X [retrieved on 1993-12-30]**
- **Yuqing Miao ET AL: "Using electropolymerized non-conducting... (PDF Download Available)", , 1 May 2004 (2004-05-01), XP055473131, Retrieved from the Internet: URL:https://www.researchgate.net/publication/271895526_Using_electropolymerized_non-conducting_polymers_to_develop_enzyme_ampe rometric_biosensors [retrieved on 2018-05-07]**
- **ZHANG S ET AL: "Construction of covalently attached enzyme multilayer films based on the photoreaction of diazo-resins and glucose oxidase", ELECTROCHIMICA ACTA, ELSEVIER SCIENCE PUBLISHERS, BARKING, GB, vol. 49, no. 26, 15 October 2004 (2004-10-15), pages 4777-4786, XP027193661, ISSN: 0013-4686 [retrieved on 2004-07-25]**
- **PÖLLER SASCHA ET AL: "Low potential biofuel cell anodes based on redox polymers with covalently bound phenothiazine derivatives for wiring flavin adenine dinucleotide-dependent enzymes", ELECTROCHIMICA ACTA, vol. 110, 27 February 2013 (2013-02-27), pages 152-158, XP028767582, ISSN: 0013-4686, DOI: 10.1016/J.ELECTACTA.2013.02.083**
- **J. DUMONT ET AL: "Behavior of glucose oxidase immobilized in various electropolymerized thin films", BIOTECHNOLOGY AND BIOENGINEERING, vol. 49, no. 5, 5 March 1996 (1996-03-05), pages 544-552, XP055493138, US ISSN: 0006-3592, DOI: 10.1002/(SICI)1097-0290(19960305)49:5<544: :AID-BIT7>3.0.CO;2-J**
- **Clément Maerten: "Morphogen Electrochemically Triggered Self-Construction of Polymeric Films Based on Mussel-Inspired Chemistry - Langmuir (ACS Publications)", Langmuir, 15 December 2015 (2015-12-15), pages 13385-13393, XP055493110, Retrieved from the Internet: URL:https://pubs.acs.org/doi/10.1021/acs.langmuir.5b03774 [retrieved on 2018-07-17]**
- **GAULTHIER RYDZEK ET AL: "Electrochemically Triggered Assembly of Films: A One-Pot Morphogen-Driven Buildup", ANGEWANDTE CHEMIE INTERNATIONAL EDITION, vol. 50, no. 19, 14 April 2011 (2011-04-14), pages 4374-4377, XP055493112, ISSN: 1433-7851, DOI: 10.1002/anie.201007436**
- **Clément Maerten ET AL: "Review of Electrochemically Triggered Macromolecular Film Buildup Processes and Their Biomedical Applications", ACS applied materials & interfaces, vol. 9, no. 34, 30 August 2017 (2017-08-30), pages 28117-28138, XP055704878, US ISSN: 1944-8244, DOI: 10.1021/acsami.7b06319**

## Description

### TECHNICAL FIELD

The present invention relates to a liquid composition comprising a protein, a polyphenol and a morphogen; where the protein, the polyphenol and the morphogen are as characterised in the appended set of claims;

to a solid composition comprising the cross-linked product of a protein and a polyphenol; where the protein and the polyphenol are as characterised in the appended set of claims;
to a biosensor and a biofuel cell comprising said solid composition bound to a surface of an electrochemical probe; to a process for the detection of an analyte with said biosensor; and to the use of a ferrocene as a morphogen in an electrodeposition process.

### BACKGROUND OF THE INVENTION

The integration of biomolecules, such as proteins and antibodies, onto electronic platforms is used to obtain functional bioelectronic devices for the detection of biomarkers. In biomedical applications, proteins are commonly used as recognition elements due to their high specificity, selectivity and catalyst efficiency in physiological conditions. Protein-based biosensors are based on the use of proteins to transduce biorecognition as electric output signals. In medical diagnosis and environmental-monitoring, protein-based biosensors are widely applied due to their high sensitivity, potential selectivity, and their possibility of miniaturization/automation.
Protein-based biosensors are a combination of an electrochemical probe, such as an electrode, having an active protein immobilized on a surface thereof. An extensive variety of redox enzymes, such as glucose oxidase (GOX), horseradish peroxidase, lactate oxidase, alcohol dehydrogenase, aldehyde dehydrogenase or urease, have been used for the elaboration of glucose, $H_2O_2$, lactate, ethanol, aldehyde or urea biosensors, respectively. Enzymatic micro-biosensors have also been developed for short-term brain applications in animal models to monitor neurotransmitters, such as glutamate or choline, using immobilized glutamate or choline oxidase, respectively. GOX biosensors have been extensively developed due to their ability to detect blood glucose and their effectiveness in the diagnostic analysis of diabetes. A representative case of success is the glucometer with test strips.
Protein immobilization is a critical process in the development of biosensors, as is the necessity to avoid their denaturation and ensure their accessibility towards the analyte.
Different techniques have been implemented for the immobilization of proteins onto solid surfaces, including adsorption, covalent binding, entrapment, cross-linking or affinity. However, protein adsorption and entrapment exhibit undesirable protein leaking issues. Covalent binding and cross-linking are able to increase enzyme stability but often exhibit reduced activity. The lack of accuracy of the systems obtained with these known techniques is a major concern. Low accuracy of the fabrication protocols, high sensitivity of proteins to the immobilization protocol or environmental factors can affect the reliability and reproducibility of glucose measurements.
Electrodeposition of macromolecules is increasingly considered to be the most suitable method for the design of biosensors. Indeed, said method is a simple and attractive bottom-up approach and can be used to finely control the immobilization of a protein on a surface of an electrode with an electrical stimulus. Conventional electrodeposition processes used to develop protein-based biosensors can be divided into three main categories:

(i) precipitation of polyelectrolytes or proteins through change of solubility,
(ii) self-assemblies of polyelectrolytes through electrostatic/ionic interactions; and
(iii) formation of covalent bonds between monomers (electropolymerization).

Another recent electrodeposition method is the electro-cross-linking process. Protein immobilization by electro-cross-linking is more efficient than conventional manual cross-linking by drop- or dip-coating of liquid protein preparations containing suitable crosslinkers, such as glutaraldehyde. Indeed, electro-cross-linking offers better reproducibility and better control of the immobilization process and can be used for the development of miniaturized biosensors through the functionalization of specific electrodes out of an assorted microelectrode array.
The Applicants have developed a concept named morphogenic electrotriggered self-construction of films, based on electro-cross-linking between two polymers in one pot using a morphogen. A morphogen is a molecule or an ion that is produced at an interface and diffuses into the solution, thus creating a concentration gradient and locally inducing a chemical reaction or interaction between two non-interacting species.
Rydzek, G. et al. "Electrochemically Triggered Assembly of Films: A One-Pot Morphogen-Driven Buildup", Angew. Chemie - Int. Ed., 2011, 50, 4374-4377 discloses the use of Cu(I) as a morphogen to obtain polymeric films using a Cu(I)-catalyzed click reaction between azides and alkynes. However, said method is not adapted to the electro-cross-linking of proteins since proteins do not comprise alkyne or azide functional groups.

Maerten, C. et al. "Electrochemically Triggered Self-Construction of Polymeric Films Based on Mussel-Inspired Chemistry", Langmuir, 2015, 31, 13385-13393 discloses the electro-cross-linking of polyamines based on mussel-inspired chemistry using a homobifunctional catechol ethylene oxide molecule, named bis-catechol, as a cross-linker and a morphogen. Indeed, the exceptional ability of mussels to adhere on almost any type of surfaces is based on catechol biochemistry (*i.e.* hydrogen bonds, metal-ligand complexes and covalent bond formation). In particular, when oxidized into the corresponding quinone, a catechol is able to react with a nucleophilic group, such as an amine or thiol, through Michael addition and Schiff's base formation. However, when said method was applied to the electro-cross-linking of enzymes, the resulting film did not exhibit any enzymatic activity and could therefore not be used in the preparation of a biosensor for the detection of an analyte.

Siebert et al. (Journal of Agricultural and Food Chemistry, vol. 47, no. 2, 1999) discloses the effects of protein and polyphenol interactions on beverage haze, such as in beer.

US 2003/104119 discloses a method of depositing an enzyme on an electrically conductive substrate. It discloses the use of a monomer in the electropolymerisation step, such as phenol, 4-aminophenol, 1,4-dihydroxybenzene, 1,3-dihydroxybenzene, 1,2-dihydroxybenzene, 3-hydroxytyramine, 1,3,5-trihydroxybenzene, and 1,2,3-trihydroxybenzene.

Eddy et al. (Biosensors and Bioelectronics, vol. 10, no. 9/10, 1994) discloses the modification of enzyme electrode properties with non-conducting electropolymerised films. It discloses that phenol and several phenol derivatives, such as phenol red and 4-aminophenol have been electropolymerised on to platinum anodes.

Bartlett et al. (Biosensors, vol. 3, no. 6, 1987) discloses strategies for the development of amperometric enzyme electrodes. It discloses immobilization of glucose oxidase using the following monomers: pyrrole, N-methyl pyrrole, aniline or phenol.

Bartlett et al. (Journal of Electroanalytical Chemistry and Interfacial Electrochemistry, vol. 362, no. 1-2, 1993) discloses the immobilization of enzymes in electropolymerised films.

Yuqing Miao et al. (:https://www.researchgate.net/publication/271895526_Using_ electropolymerized_non-conducting_polymers_to_develop_enzyme_amperometric_ biosensors) discloses the use of electropolymerised non-conducting polymers to develop enzyme amperometric biosensers.

Zhang et al. (Electrochimica Acta, vol. 49, no. 26, 2004) discloses the construction of covalently attached enzyme multilayer films based on the photoreaction of diazo-resins (DAR) and glucose oxidase (GOX).

Pöller et al. (Electrochimica Acta, vol. 110, 2013) discloses the synthesis of a variety of redox polymers based on phenothiazine derivatives.

Dumont et al. (Biotechnology and Bioengineering, vol. 49, no. 5, 1996) discloses immobilization of glucose oxidase in various electropolymerised thin films using aniline, indole, pyrrole and phenylediamine.

After extensive research, Applicants have developed a one-pot electrotriggered self-construction of protein-based films for second-generation biosensors by contacting an electrochemical probe with a liquid composition comprising a protein, a polyphenol and a morphogen; and applying an electric stimulus to the electrochemical probe so as to form a solid composition comprising the cross-linked product of an protein and a polyphenol on a surface of the electrode. The protein is covalently bound to the surface of the electrochemical probe and is not prone to leaking. The biosensor obtained with said solid composition allows the electrochemical detection of an analyte, such as glucose, with excellent sensitivity and selectivity. Further, the electro-cross-linking process of the invention is regioselective and can be used to develop miniaturized biosensors through functionalization of specific electrodes out of a microelectrode array.

## SUMMARY OF THE INVENTION

The invention is set out in the appended set of claims.

A first object of the invention is a liquid composition as in the appended claims comprising a protein, a polyphenol, and a morphogen.

Another object of the invention is a solid composition as in the appended claims comprising a cross-linked product of a protein and a polyphenol.

Yet another object of the invention is a process for the preparation of the solid composition of the invention, wherein the process comprises the steps of:

- contacting an electrochemical probe with the liquid composition of the invention;
- applying an electric stimulus to the electrochemical probe so as to form a solid composition comprising a cross-linked product of a protein and a polyphenol on a surface of the electrochemical probe.

The present invention is also directed to a biosensor comprising an electrochemical probe, wherein a solid composition comprising a cross-linked product of a protein and a polyphenol is bound to a surface thereof.

Further the present invention also aims at providing a process for the detection of an analyte in a sample, wherein the process comprises the steps of:

- contacting the biosensor of the invention with a solution containing a mediator in a housing;
- applying a current, an electric potential or an inductance to obtain the mediator in reduced form;
- introducing a sample containing an analyte in the housing;
- measuring the variation of electric signal generated by the oxidation of the mediator.

Another object of the invention is a biofuel cell comprising:

- a positive electrode;
- a negative electrode;
- an electrolyte; and
- an external circuit electrically connecting the positive electrode and the negative electrode;

wherein a solid composition comprising a cross-linked product of a protein and a polyphenol is bound to a surface of the positive electrode and/or the negative electrode.

A final object of the invention is the use of a ferrocene as a morphogen in the formation of a solid composition on the surface of an electrode by electrodeposition.

## DESCRIPTION OF FIGURES

**Figure 1** shows the principle of the process for the preparation of a solid composition of the invention based on three steps: (1) electro-oxidation of a morphogen (methanol ferrocene in the figure) which creates a gradient of oxidized morphogen (methanol ferrocenium in the figure), by application of a cyclic voltammetry, (2) oxidation of polyphenol (bis-catechol in the figure) into bis-quinone molecules by reaction with the oxidized morphogen and (3) chemical reaction of bis-quinone with free amino moieties of the protein (GOX in the figure), through Michael addition and Schiff's base condensation reaction.

**Figure 2** shows the cyclic voltammograms, performed at a scan rate of 0.05 V/s, of (a) a solution of polyphenol of formula (III) (6.1 mmol/L) and (b) a solution comprising a mixture of polyphenol of formula (III) (6.1 mmol/L) and methanol ferrocene (0.5 mmol/L), the arrows indicate the evolution of the signal during the cyclic voltammetry (CV) application and (c) the evolution of the normalized frequency shift, measured by EC-QCM, as a function of time of polyphenol of formula (III) (black curve) and polyphenol/ferrocene mixture (gray curve) solutions during the application of CV between -0.4 and 0.7 V (scan rate 0.05 V/s). The supporting electrolyte was phosphate buffer solution at pH 7.4.

**Figure 3** shows the evolution of (a) the normalized frequency shift, measured at 15 MHz by QCM, as a function of time during the self-construction of GOX/polyphenol film of example 4 by application of cyclic voltammetry (0-0.7 V, scan rate 0.05 V/s) for 45 min using a solution comprising a mixture of polyphenol of formula (III), GOX and methanol ferrocene in phosphate buffer and (b) measured cyclic voltammograms, first five cycles (black curve) and last five cycles (gray curve), the arrows indicate the evolution of the signal during the self-construction.

**Figure 4** shows the typical topographic atomic force microscopy (AFM) images obtained in contact mode and liquid state, before ($50 \times 50\ \mu m^2$, z-scale = 400 nm), and after scratching ($25 \times 25\ \mu m^2$, z-scale = 300 nm) with respective cross-section profiles of GOX/polyphenol films of example 4 obtained after (a) 15 min, (b) 30 min, (c) 45 min and (d) 60 min of self-construction. The white dotted bars represent the cross-section area.

**Figure 5** shows the evolution of the thickness and the roughness, measured by AFM in contact mode and liquid state, of self-constructed GOX/polyphenol films of example 4 as a function of self-construction time. The roughness was calculated on $3 \times 3\ \mu m^2$ AFM images.

**Figure 6** shows (a) Schematic representation of GOX catalytic reactions and oxido-reduction of ferrocene methanol in a self-constructed GOX/polyphenol film of example 4 allowing glucose sensing and (b) Cyclic voltammograms, performed at scan rate of 0.05 V/s, of a self-constructed GOX/polyphenol film of example 4 in contact with pure 10 mmol/L phosphate buffer saline (- -), 10 mmol/L glucose (-), 0.5 mmol/L ferrocene methanol (- • -), 10 mmol/L glucose and 0.5 mmol/L ferrocene methanol (- - - - - -), 20 mmol/L glucose and 0.5 mmol/L ferrocene methanol (•••), 40

mmol/L glucose and 0.5 mmol/L ferrocene methanol (- •• -) solutions, prepared in 10 mmol/L phosphate buffer saline, measured in Ar-saturated environment.

**Figure 7** shows the typical steady-state current response of a self-constructed GOX/polyphenol film of example 4 upon addition of 600 μL of different concentrations of glucose in the presence of 0.5 mmol/L ferrocene methanol during the application of +0.25 V.

**Figure 8** shows (a) Typical steady-state current response of self-constructed GOX/polyphenol film of example 4 upon addition of 600 μL of different interfering substances, ascorbic acid (AA), uric acid (UA), salicylic acid (SA) and acetaminophen (AP) in the absence and in the presence of 5 mmol/L glucose during the application of +0.25 V in the presence of 0.5 mmol/L ferrocene methanol (b) Histogram of the current density of the film depending on the nature of the interfering species, with 5 mmol/L glucose and the interfering species (black) and interfering species in the absence of glucose (gray).

**Figure 9** shows the evolution of the absorbance, measured at 440 nm, as a function of time of o-dianisidine, horseradish peroxidase (HRP) and glucose mixture in contact with GOX/polyphenol films of example 4 self-constructed (a) for 15 min (•), 30 min (▼), 45 min (□) and 60 min (0) (b) Leaking test where the absorbance was followed in the presence of the GOX/polyphenol film of example 4, self-constructed for 30 min, followed by its removal from the supernatant. GOX's activity is monitored by using a second enzyme, HRP, which will use the $H_2O_2$ produced by GOX enzymatic reaction to react with o-dianisidine (colourless) to obtain the o-dianisidine oxidized, which is brown. The reaction was followed at 440 nm. The measurements were performed with a microplate reader.

**Figure 10** shows the typical steady-state current response of self-constructed GOX/polyphenol film of example 4 during the application of +0.25 V upon addition of 600 μL of different concentrations of glucose in the presence of 0.5 mmol/L of methanol ferrocene followed by three sequential washes with 0.01% of Tween® 20 detergent.

**Figure 11** shows (a) an interdigitated array (IDA) of electrodes having a width of 10 μm, each electrode being separated by 5 μm and (b) schematic representation of the IDA electrodes with one of the array electrodes used as the working electrode (grey) and the counter electrode (dark grey). The IDA electrodes were used in a 3-electrode electrochemical set-up with a no-leak Ag/AgCl as reference electrode dipped in the solution.

**Figure 12** shows the optical microscope images of interdigitated array (IDA) of electrodes of example 7 (a) in fluorescence mode and (b) in bright field where microelectrodes within an IDA were addressed to form a cross-linked product of polyphenol of formula (III) and rhodamine-labeled GOX ($GOX^{Rho}$) by application of cyclic voltammetry (0-0.7 V, scan rate 0.05 V/s) for 45 min on one of the two arrays using a solution comprising a mixture of polyphenol, $GOX^{Rho}$ and methanol ferrocene in phosphate buffer in contact with the IDA. The scale bar represents 25 μm.

## DETAILED DESCRIPTION

As used herein, the singular forms "a", "an" and "the" are intended to include the plural forms as well, unless the context clearly indicates otherwise.

Additionally, the use of "or" is intended to include "and/or", unless the context clearly indicates otherwise.

As used herein, the term "($C_1$-$C_6$)alkyl" refers to a straight or branched saturated hydrocarbon having 1 to 6 carbon atoms. Examples of such groups include, but are not limited to, methyl, 2-propyl, 1-butyl, 2-butyl, 2-methyl-2-propyl, 2-methyl-1-butyl and 1-hexyl.

As used herein, the term "($C_2$-$C_6$)alkene" refers to a refers to a straight or branched unsaturated hydrocarbon having 1 to 6 carbon atoms.

As used herein, the term "($C_1$-$C_6$)hydroxyalkyl" refers to a ($C_1$-$C_6$)alkyl that is substituted with at least one hydroxy (-OH) group.

As used herein, the term "($C_1$-$C_6$)haloalkyl" refers to a"($C_1$-$C_6$)alkyl that is substituted with at least one halogen atom, such as Cl, Br, I or F.

As used herein, the term "azide" refers to a $-N_3$ group.

As used herein, the term "formyl" refers to a -CHO group.

As used herein, the term "carboxyl" refers to a -COOH group.

As used herein, the term "($C_1$-$C_6$)alkanoic acid" refers to a ($C_1$-$C_6$)alkyl that is substituted with at least one carboxyl (-COOH) group.

As used herein, the term "($C_1$-$C_6$)aminoalkyl" refers to a ($C_1$-$C_6$)alkyl that is substituted with at least one amino

($-NH_2$) group.

As used herein the term "($C_1$-$C_6$)alkylamino" refers to a $-NHR_a$ group wherein $R_a$ is a ($C_1$-$C_6$)alkyl group.

As used herein the term "di(($C_1$-$C_6$)alkyl)amino" refers to a $-NR_aR_b$ group wherein $R_a$ and $R_b$ are a ($C_1$-$C_6$)alkyl.

As used herein, the term "($C_1$-$C_6$)alkyl-($C_1$-$C_6$)alkylamino" refers to a ($C_1$-$C_6$)alkylamino group that is connected by a ($C_1$-$C_6$)alkyl radical.

As used herein, the term "($C_1$-$C_6$)alkyl-di(($C_1$-$C_6$)alkyl)amino" refers to a di(($C_1$-$C_6$)alkyl)amino group that is connected by a ($C_1$-$C_6$)alkyl radical.

As used herein, the term "($C_1$-$C_6$)carbonylalkyl" refers to a ($C_1$-$C_6$)alkyl group that is connected by a carbonyl (C=O) group.

As used herein, the term "($C_1$-$C_6$)carboxyalkyl" refers to a ($C_1$-$C_6$)carbonylalkyl wherein the carbonyl group is connected by an oxygen bridge.

As used herein, the term "($C_1$-$C_6$)alkyl($C_1$-$C_6$)carboxyalkyl" refers to a "($C_1$-$C_6$)carboxyalkyl that is connected by a ($C_1$-$C_6$)alkyl radical.

As used herein, the term "($C_1$-$C_6$)carbonylhaloalkyl" refers to a ($C_1$-$C_6$)haloalkyl group that is connected by a carbonyl (C=O) group.

## Liquid composition

The liquid composition of the invention comprises a protein, a polyphenol and a morphogen.

As used herein, the term "liquid composition" refers to a composition that flows under its own weight within a temperature range of 5°C to 50°C.

In particular, the liquid composition may be an aqueous composition. The term "aqueous composition" refers to a composition that comprises water. The aqueous composition may further optionally comprise a non-aqueous solvent.

More particularly, the liquid composition of the invention may be an aqueous solution. The term "solution" refers to homogenous liquid composition in which the different constituents, namely the protein, the polyphenol and the morphogen, are dissolved.

The liquid composition of the invention may be a buffered solution. As used herein, the term "buffered solution" relates to a solution comprising a buffering agent. Buffering agents may be weak acids or bases used to maintain the acidity (pH) of a solution near a chosen value even when further acids or bases are added. In the present invention, the oxidation of the polyphenol during the electro-cross-linking process generates H+ ions. The presence of a buffering agent in the liquid composition of the invention advantageously prevents the pH from becoming too acidic which is detrimental for the reactivity of the amine functions of the protein.

Preferably, the liquid composition of the invention may be a phosphate buffered solution. The liquid composition may, for example, comprise 50 to 250 mmol, in particular 100 to 200 mmol, more particularly 120 to 170 mmol, of phosphate per liter of solution. The liquid composition may further comprise NaCl and/or KCl. For example, the liquid composition may comprise 1 to 3 mol, in particular, 1.5 to 2.5 mol, more particularly 1.8 to 2.2 mol of NaCl per liter of solution. The liquid composition may also comprise 0.01 to 0.1 mol, in particular, 0.02 to 0.6 mol, more particularly 0.3 to 0.5 mol, of KCl per liter of solution.

The liquid composition of the invention may exhibit a pH of 5 to 9, in particular 6 to 8, more particularly 7 to 7.8.

In the liquid composition of the invention, the protein and the polyphenol are in their free form, i.e. they are not covalently linked one with another.

The liquid composition may be flushed with nitrogen so as remove dissolved oxygen in order to prevent oxidation of the polyphenol.

## Protein

The liquid composition of the invention comprises a protein.

As used herein, the term "protein" refers to a polymer of amino acids joined together by peptide bonds. Proteins perform a vast array of functions within organisms, including catalyzing metabolic reactions, DNA replication, responding to stimuli, and transporting molecules from one location to another. Proteins differ from one another primarily in their sequence of amino acids, which is dictated by the nucleotide sequence of their genes. Proteins usually exhibit a three-dimensional structure that determines its activity.

The protein may be an enzyme or an antibody.

In one embodiment, the protein may be an enzyme.

As used herein, the term "enzyme" refers to a biological macromolecule that is capable of catalyzing biochemical reactions. Generally, enzymes are proteins, such as globular proteins. The enzyme may optionally be associated with a cofactor. As used herein, the term "cofactor" refers to a non-protein compound, such as an organic molecule or a metallic ion, required for an enzyme's activity.

The enzyme may be a redox enzyme. As used herein, the term "redox enzyme" refers to an enzyme that catalyzes the transfer of electrons from one molecule, the reductant, also called the electron donor, to another, the oxidant, also called the electron acceptor.

In particular, the enzyme may be selected from a dehydrogenase, a reductase, an oxidase, an oxygenase, a peroxidase, a catalase, a transhydrogenase, a transferase, a hydrolase, a lyase, an isomerase, a ligase and a urease.

More particularly, the enzyme may be selected from glucose oxidase (GOX), horseradish peroxidase (HRP), lactate oxidase, alcohol dehydrogenase, aldehyde dehydrogenase, urease, glutamate oxidase, choline oxidase, glucose dehydrogenase, laccase, bilirubin oxidase, ascorbate oxidase, formate dehydrogenase, lactate dehydrogenase, pyruvate dehydrogenase, malate dehydrogenase, p-cresolmethylhydroxylase, methylamine dehydrogenase, succinate dehydrogenase, fumarate reductase, D-fructose dehydrogenase, D-gluconate dehydrogenase, cytochrome c, peroxidase, ferredoxin, plastocyanin, azurin, and azotoflavin.

Even more particularly, the enzyme may be GOX.

In another embodiment, the protein may be an antibody.

As used herein, the term "antibody" refers to a protein that is produced by specialized B cells after stimulation by an antigen, such as a pathogenic bacteria or a virus, and acts specifically against the antigen in an immune response.

In particular, the antibody may be an immunoglobulin, for example immunoglobulin G (IgG).

The protein may optionally be labeled with a fluorescent tag, such as rhodamine, so as to monitor the localization of the protein by fluorescence.

The liquid composition may comprise from 0.5 to 100 mmol, in particular 1 to 50 mmol, more particularly 5 to 10 mmol, of protein per liter of liquid composition.

**Polyphenol**

The liquid composition of the invention comprises a polyphenol. As described herein, but not being part of the claimed invention the term "polyphenol" refers to a molecule comprising more than one phenyl group, preferably 2 to 10 phenyl groups, wherein each phenyl group is substituted by more than one hydroxy group, preferably by two or three hydroxy groups. The polyphenol is capable of being oxidized into the corresponding quinone by application of a potential with an electrode. In particular, the oxidized polyphenol is able to establish cross-links between proteins and cross-links between proteins and the surface of the electrode.

In particular, the polyphenol may correspond to the following formula (I):

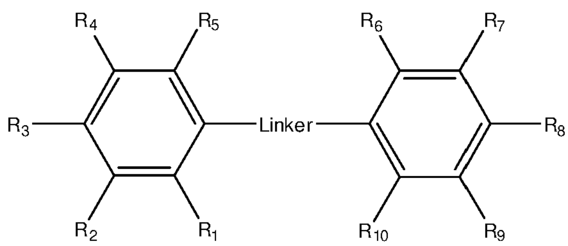

(I)

wherein $R_1$-$R_{10}$ are each independently selected from H and OH provided that at least two of $R_1$-$R_5$ are OH at least two of $R_6$-$R_{10}$ are OH;

the linker is a hydrocarbon chain optionally interrupted by one or more heteroatoms selected from N, O and S, wherein the hydrocarbon chain is optionally substituted by one or more functional groups selected from carbonyl, thiocarbonyl, $C_1$-$C_8$ alkyl, halogen, - COOH; or the linker is a heteroaryl, in particular a triazole.

According to the invention, the polyphenol corresponds to the following formula (II):

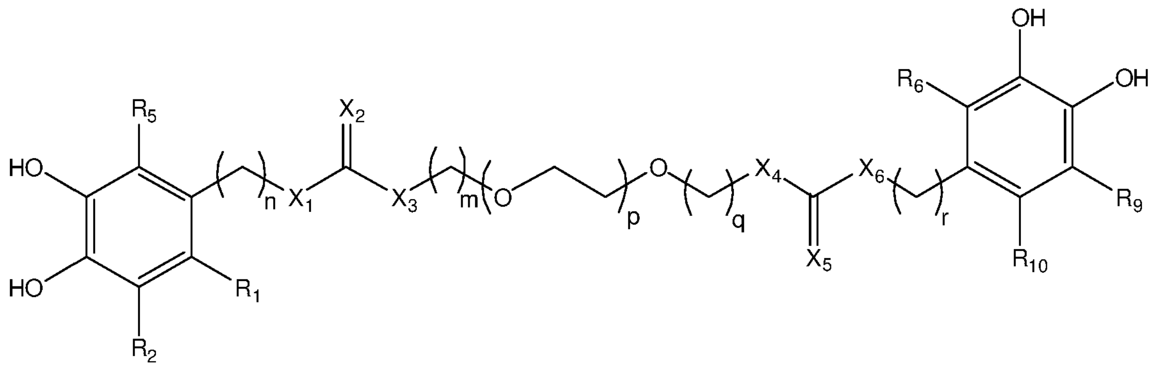

(II)

wherein $R_1$, $R_2$, $R_5$, $R_6$, $R_9$, and $R_{10}$ are each independently selected from H and OH;
$X_1$, $X_3$, $X_4$ and $X_6$ are each independently a bond, O, N or S;
$X_2$ and $X_5$ are each independently O or S;
n, m, p, q are r are integers that are independently equal to 0, 1, 2, 3, 4, 5, 6, 7 or 8, provided that n, m, p, q are r are not all equal to 0.

Even more particularly, the polyphenol may correspond to the following formula (III):

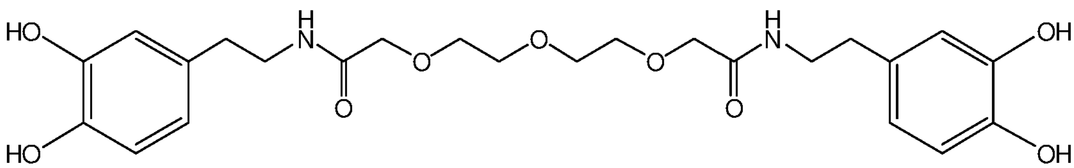

(III).

The liquid composition may comprise from 0.5 to 100 mmol, in particular 1 to 50 mmol, more particularly 5 to 10 mmol, of polyphenol per liter of liquid composition.

In one embodiment, the amount of polyphenol in the composition is determined as a function of the number of accessible amine groups of the protein, i.e. the number of amine groups on the protein that are able to react with a quinone through Michael addition and Schiff's base condensation reaction. In particular, the liquid composition may exhibit a [phenol]/[amine] molar ratio of 0.05 to 0.5, in particular 0.08 to 0.4, more particularly 0.1 to 0.3, wherein [phenol] corresponds to the number of phenyl groups bearing OH substituents of the polyphenol, and [amine] corresponds to the number of accessible amine groups of the protein.

**Morphogen**

The liquid composition of the invention comprises a morphogen. As described herein, but not being part of the claimed invention the term "morphogen" refers to a molecule or ion that is capable of being oxidized by application of a potential with an electrode. In particular, the oxidized morphogen is able to induce cross-linking reactions between the protein and the polyphenol and between the protein, the polyphenol, and the surface of the electrode. The morphogen is thus distinct from the polyphenol and the protein.

The morphogen is selected from a ferrocene, a source of protons, a ruthenium complex and a ferricyanide complex, a source of hydroxide, a nickelocene, an osmium complex, an iron complex, a cobalt complex, methylene blue, dihydroxybenzoquinone, manganese cyclopentadienyl and an oxidized viologen.

According to the invention, the morphogen is a ferrocene. As used herein, the term "ferrocene" refers to an organometallic chemical compound consisting of two five-membered rings bound on opposite sides of a central iron atom. The five-membered rings of the ferrocene comprise 5 ring atoms selected from C, N, P, preferably 5 carbon ring atoms. Further, each five-membered ring may be independently substituted by 1 to 5 substituents and/or may be fused with another 6-membered ring. The substituents of the five-membered ring may each be independently selected from $(C_1-C_6)$alkyl, $(C_2-C_6)$alkene, $(C_1-C_6)$hydroxyalkyl, $(C_1-C_6)$haloalkyl, azide, formyl, carboxyl, $(C_1-C_6)$alkanoic acid, $(C_1-C_6)$aminoalkyl, $(C_1-C_6)$alkyl-$(C_1-C_6)$alkylamino, $(C_1-C_6)$alkyl-di($(C_1-C_6)$alkyl)amino, $(C_1-C_6)$carbonylalkyl, $(C_1-C_6)$carboxyalkyl, $(C_1-C_6)$alkyl$(C_1-C_6)$carboxyalkyl and $(C_1-C_6)$carbonylhaloalkyl.

In particular, the ferrocene may comprise two cyclopentadienyl rings and may be a 1-substituted ferrocene, i.e. a ferrocene comprising one substituent on one ring; or a 1,1'-disubstituted ferrocene i.e. a ferrocene comprising one substituent on each ring, wherein the one or two substituents are independently selected from ($C_1$-$C_6$)alkyl, ($C_2$-$C_6$)alkene, ($C_1$-$C_6$)hydroxyalkyl, ($C_1$-$C_6$)haloalkyl, azide, formyl, carboxyl, ($C_1$-$C_6$)alkanoic acid, ($C_1$-$C_6$)aminoalkyl, ($C_1$-$C_6$)alkyl-($C_1$-$C_6$)alkylamino, ($C_1$-$C_6$)alkyl-di(($C_1$-$C_6$)alkyl)amino, ($C_1$-$C_6$)carbonylalkyl, ($C_1$-$C_6$)carboxyalkyl, ($C_1$-$C_6$)alkyl($C_1$-$C_6$)carboxyalkyl and ($C_1$-$C_6$)carbonylhaloalkyl.

More particularly, the ferrocene may be selected from di(cyclopentadienyl)iron, methanol ferrocene, acetylferrocene, 1,1'-diacetylferrocene, (dimethylaminomethyl)ferrocene, ferrocenecarboxaldehyde, (1-acetoxyethyl)ferrocene, ferrocenoyl azide, $\alpha$-methylferrocenemethanol, 1-(dimethylamino)ethyl]ferrocene, aminomethylferrocene, 1,1'-di(aminomethyl)ferrocene, aminoethylferrocene, 1,1' -di(aminoethyl)ferrocene, ferrocenecarboxylic acid, 1,1'-ferrocenedicarboxylic acid, and (6-Bromo-1-oxohexyl)ferrocene.

Even more particularly, the morphogen may be methanol ferrocene.

The liquid composition may comprise from 0.01 to 10 mmol, in particular 0.05 to 5 mmol, more particularly 0.1 to 1 mmol, of morphogen per liter of liquid composition.

## Solid composition

The solid composition of the invention comprises the cross-linked product of a protein and a polyphenol, in particular the cross-linked product of an enzyme and a polyphenol.

As used herein, the term "solid composition" refers to a composition that does not flow under its own weight within a temperature range of 5°C to 50°C.

As used herein, the term "cross-linked product of a protein and a polyphenol" refers to a copolymer comprising two different monomeric units derived from a protein and a polyphenol. The proteins are covalently cross-linked with one another by the polyphenols. Indeed, in its oxidized state, the polyphenol exhibits an affinity for the amino groups of the protein. The polyphenol thus acts as a cross-linking agent between the proteins, in particular by binding with some of the amino groups of the proteins.

The protein and the polyphenol in the cross-linked product of the solid composition may be as defined hereinabove. In one embodiment, the solid composition of the invention does not comprise ferrocene. More particularly, the solid composition consists essentially of the cross-linked product of a protein and a polyphenol.

In one embodiment, the solid composition of the invention may be in the form of a film. As used herein, the term "film" refers to tridimensional material in the form of a thin layer.

More particularly, the solid composition may be in the form of a film bound to the surface of an electrochemical probe. As used herein, the term "film bound to the surface of' refers to a layer of solid composition covalently bound to the surface of the electrochemical probe. As such, the present invention is not directed to the physical deposition of the protein on the surface of the electrochemical probe or to the physical immobilization or the protein by embedding the protein in a matrix, such as a paint.

Even more particularly, the solid composition may be bound to the surface of the electrochemical probe by means of the polyphenol. Indeed, in its oxidized state, the polyphenol exhibits an affinity for the surface of the electrode and the amino groups of the protein. The polyphenol thus acts as a cross-linking agent between the protein and the surface of the electrode, in particular by binding with some of the amino groups of the protein and the surface of the electrode.

In one embodiment, the solid composition of the invention is in the form of a film that exhibits a thickness of 40 to 150 nm, in particular 45 to 120 nm, more particularly 50 to 100 nm. The thickness of the film may be measured according to the Thickness Test Method described herein.

Further, the solid composition of the invention may be in the form of a film comprising the cross-linked product of an enzyme and a polyphenol that exhibits an enzymatic activity ( $K_m^{app}$ ) that is lower than 100 mmol/L, in particular lower than 20 mmol/L, more particularly lower than 10 mmol/L, even more particularly lower than 7 mmol/L. The enzymatic activity ( $K_m^{app}$ ) may be determined according to the Electrochemical Enzymatic Activity Test Method disclosed herein.

The solid composition of the composition may exhibit a percentage of enzyme leaking of less than 5%, in particular less than 1%, more particularly 0%, wherein the percentage of enzyme leaking corresponds to the molar percentage of immobilized enzyme that is removed from the solid composition by washing with a detergent according to the Enzyme Leaking Test Method disclosed herein.

The solid composition of the invention may be obtained by applying an electric stimulus to an electrochemical probe in contact with the liquid composition of the invention. The process for the preparation of said solid composition is described hereinafter.

## Process for the preparation of a solid composition

The process for the preparation of the solid composition of the invention comprises the step of contacting an electrochemical probe with the liquid composition of the invention. The liquid composition is as defined herein above.

The electrochemical probe may an amperometric, voltammetric or conductimetric electrochemical probe. In particular, the electrochemical probe may be an electrode, more particularly a working electrode.

The electrochemical probe preferably exhibits an affinity for the polyphenol. Examples of suitable materials for the electrochemical probe in the context of the invention include gold, platinum, silver, copper, palladium, iridium, ruthenium, aluminum, nickel, titanium, indium tin oxide (ITO), zinc oxide (ZnO), iron and carbon (graphite, graphene, carbon nanotubes); preferably gold. In another embodiment, the electrochemical probe may be a screen printed electrode.

The process for the preparation of the solid composition of the invention further comprises the step of applying an electric stimulus to the electrochemical probe so as to form a solid composition comprising the cross-linked product of a protein and a polyphenol on a surface of the electrochemical probe.

The electric stimulus should be selected so as to induce electro-oxidation of the morphogen and create a gradient of oxidized morphogen from the surface of the electrochemical probe. The oxidized morphogen will then oxidize the phenol groups of the polyphenol into quinone groups which will in turn enable the chemical reaction of the quinone groups with free amino moieties of the protein through Michael addition and Schiff's base condensation reaction.

In one embodiment, the application of an electric stimulus may be the application of cyclic voltammetry, for example between -2 and 1.5 V, in particular between 0 and 0.7 V, with a scan rate of 0.01 to 0.2 V/s, in particular 0.05 V/s.

The process of the invention may be conducted in a 3-electrode cell comprising a working electrode, a counter-electrode and a reference electrode. The working electrode is immersed in the liquid composition of the invention and corresponds to the electrochemical probe on which the film of solid composition is formed. The counter-electrode may be a platinum electrode. The reference electrode may be a Ag/AgCl electrode.

The electric stimulus may be applied for a period of time sufficient to form a film of solid composition having the desired thickness. In particular, the period of time may be 1 to 120 minutes, in particular 15 to 90 minutes, more particularly 30 to 60 minutes, even more particularly 45 minutes.

After application of the electric stimulus, the process of the present invention may comprise a rinsing step by contacting the electrochemical probe with an aqueous solution, in particular a buffered solution, more particularly a phosphate buffered solution.

## Use of ferrocene as a morphogen

The present invention is also directed to the use of a ferrocene as a morphogen in the formation of a solid composition on the surface of an electrode by electrodeposition.

The ferrocene for use as a morphogen may be as described above. In particular, the ferrocene may be methanol ferrocene.

In particular, the ferrocene is used to form of a solid composition on the surface of an electrode by electrodeposition by applying an electric stimulus to an electrochemical probe so as to form a solid composition on a surface of the electrochemical probe. The solid composition obtained by electrodeposition may result from one of the following processes:

(i) precipitation of polyelectrolytes or proteins through change of solubility,
(ii) self-assemblies of polyelectrolytes through electrostatic/ionic interactions;
(iii) formation of covalent bonds between monomers (electropolymerization); or
(iv) formation of cross-links between polymer chains (electro-cross-linking).

## Biosensor and process for the detection of an analyte

The biosensor of the invention comprises an electrochemical probe.

As used herein, the term "biosensor" refers to a self-contained analytical device that combines a biological component with a physicochemical device for the detection of an analyte of biological importance within a sample, such as a biological sample.

The electrochemical probe of the biosensor comprises the solid composition comprising a cross-linked product of a protein and a polyphenol according to the invention bound to a surface thereof.

The electrochemical probe may an amperometric, voltammetric or conductimetric electrochemical probe. In particular, the electrochemical probe may be an electrode, more particularly a working electrode.

The electrochemical probe preferably exhibits an affinity for the polyphenol. Examples of suitable materials for the electrochemical probe in the context of the invention include gold, platinum, silver, copper, palladium, iridium, ruthenium, aluminum, nickel, titanium, indium tin oxide (ITO), zinc oxide (ZnO) iron and carbon (graphite, graphene, carbon nanotubes); preferably gold. In another embodiment, the electrochemical probe may be a screen printed electrode.

In one embodiment, the biosensor comprises a housing. The housing may comprise a solution containing a mediator.

As used herein, the term "mediator" refers to a molecule or ion that is capable of being oxidized by application of a potential with an electrode. The mediator advantageously exhibits an adequate solubility in both oxidized and reduced states for a rapid diffusion between the redox center of the protein and the electrode surface as well as a fast reaction with the reduced form of the protein. In particular, the mediator may be selected from a ferrocene, a source of protons, a ruthenium complex, a ferricyanide complex, a source of hydroxide, a nickelocene, an osmium complex, an iron complex, a cobalt complex, methylene blue, dihydroxybenzoquinone, manganese cyclopentadienyl, an oxidized viologen; more particularly the mediator may be a ferrocene.

The solution comprising the mediator is preferably a buffered solution, more preferably a phosphate buffered solution. The solution comprising the mediator may, for example, comprise 1 to 50 mmol, in particular 2 to 50 mmol, more particularly 5 to 20 mmol, of phosphate per liter of solution. The solution comprising the mediator may exhibit a pH of 5 to 9, in particular 6 to 8, more particularly 7 to 7.8.

The electrochemical probe comprising the solid composition of the invention may be immersed in the solution comprising the mediator. The sample containing the analyte to be detected may be injected in said housing.

The biosensor may further comprise a counter-electrode and optionally a reference electrode.

In one embodiment, the biosensor comprises a means to measure the electric signal variation in terms of current, electric potential or inductance in case of amperometric, voltammetric or conductimetric biosensors. Further, the biosensor may comprise a means to correlate the electric signal variation to the concentration of the analyte within the sample.

The present invention also relates to a process for the detection of an analyte in a sample with the biosensor of the invention.

The process of the invention comprises the steps of:

- contacting the biosensor of the invention with a solution containing a mediator in a housing;
- applying a current, an electric potential or an inductance to obtain the mediator in reduced form;
- introducing a sample containing an analyte in the housing;
- measuring the variation of electric signal generated by the oxidation of the mediator.

The mediator used in the process of the invention is as defined above. In one embodiment, the mediator is reduced by application of an adequate potential. The potential may be determined by cyclic voltammetry to identify the oxidation and reduction peaks. When the mediator is methanol ferrocene, the potential that is applied may be 0.25 V.

The variation of electric signal generated by the oxidation of the mediator may be the variation of intensity generated by the oxidation of the mediator.

The analyte detected by the biosensor may be a substance which is derived from a living body and which may serve as an index for a disease or health condition. The substance to be measured may be glucose (e.g., blood sugar), cholesterol, alcohol, sarcosine, fructosyl amine, pyruvic acid, lactic acid, and hydroxybutyric acid, for example. The sample that contains the analyte may be a biological sample. Examples of the biological sample include blood and urine.

## Biofuel cell

The biofuel cell of the invention comprises:

a positive electrode;
a negative electrode;
an electrolyte; and
an external circuit electrically connecting the positive electrode and the negative electrode;
wherein a solid composition comprising a cross-linked product of a protein and a polyphenol is bound to a surface of the positive electrode and/or the negative electrode.

The present invention is further detailed in the non-limiting examples below.

## MATERIALS AND TEST METHODS

### Chemicals

Alkaline Phosphatase (AP from bovine intestinal mucosa, CAS 9001-78-9), Glucose oxidase (GOX from *Aspergillus niger,* CAS 9001-37-0), paranitrophenyl phosphate liquid substrate (pNPP, P7998, CAS 4264-83-9), salicylic acid (SA, M = 138.12 g/mol, CAS 69-72-7), ascorbic acid (AA, M = 176.12 g/mol, CAS 50-81-7) sodium nitrate ($NaNO_3$, M = 84.99 g/mol, CAS 7631-99-4), potassium hexacyanoferrate(II) (M = 422.41 g/mol, CAS 14459-95-1), o-dianisidine peroxidase (M = 244.29 g/mol, CAS 119-90-4), glucose (M = 180.16 g/mol, CAS 50-99-7), phosphate buffered saline tablet (PBS, P4417), tris(hydroxymethyl)aminomethane (Tris, M = 121.14 g/mol, CAS 77-86-1) and ferrocene methanol (FC, M = 216.06 g/mol, CAS 1273-86-5) were purchased from Sigma-Aldrich. Dopamine hydrochloride was purchased from Aldrich. TUDA was purchased from Iris Biotech. All dried solvents were purchased from Acros Organics. Acetaminophen (AP, M = 151.17 g/mol, CAS 103-90-2) and uric acid (UA, M = 168.11 g/mol, CAS 69-93-2) were purchased from Alfa Aesar. All chemicals were used as received and dissolved in aqueous solution using MilliQ water (resistivity of 18.2 MS2.cm at 25 °C). Phosphate buffer solution was prepared at 150 mmol/L phosphate, 2.06 mol/L NaCl and 0.041 mol/L KCl and adjusted at pH 7.4.

### Electrochemical cell

The electrochemical cell used to prepare the solid composition by electrodeposition and measure the enzymatic activity is an Electrochemical Quartz Crystal Microbalance (EC-QCM) with Dissipation Monitoring. Q-Sense E1 apparatus from Q-Sense AB (Gothenburg, Sweden) was used to perform the electrochemical quartz microbalance (EC-QCM) experiments by monitoring the changes in the resonance frequency $f_v$ and the dissipation factor $D_v$ of an oscillating quartz crystal upon adsorption of a viscoelastic layer (v represents the overtone number, equal to 1, 3, 5 and 7). The measurements were executed at the first, third, fifth, and seventh overtones, corresponding to 5, 15, 25, and 35 MHz after the excitation of the quartz crystal at its fundamental frequency (5 MHz). The QCM measurement is sensitive to the amount of water associated with the adsorbed molecules and senses the viscoelastic changes in the interfacial material. Only the third overtone at 15 MHz is presented. Electrochemical measurements were performed on a CHI660E apparatus from CH instrument (Austin, Texas) coupled on the QCM-D (named EC-QCM) apparatus with a three electrode system: The gold-coated QCM sensor acted as working electrode. A platinum electrode (counter electrode) on the top wall of the chamber and a no-leak Ag/AgCl reference electrode fixed in the outlet flow channel were used respectively as counter and reference electrodes. Before the electrodeposition of the solid composition, in order to test the quality of the EC-QCM cell, a capacitive current in the presence of phosphate buffer and a faradic current of 1 mmol/L of potassium hexacyanoferrate (II) aqueous solution (prepared in phosphate buffer) was recorded by application of cyclic voltammetry (5 cycles at 50 mV/s between 0 and 0.7 V vs Ag/AgCl).

### NMR

$^1$H NMR spectra were recorded on Bruker Advance DPX400 (400 MHz) spectrometers.

### Film thickness and roughness

The thickness and roughness of the film is measured by Atomic Force Microscopy (AFM) on a film bound to the surface of an electrochemical probe. The film is obtained with the process of the invention as detailed in the examples below. AFM images were obtained in contact mode in liquid conditions with the Nanoscope IV from Veeco (Santa Barbara, CA). Cantilevers with a spring constant of 0.03 N/m and silicon nitride tips (model MSCTAUHW, Veeco) were used. Several scans were performed over a given surface area. These scans had to produce comparable images to ascertain that there is no sample damage induced by the tip. Deflection and height images were scanned at a fixed scan rate (1 Hz) with a resolution of 512 $\times$ 512 pixels. The film thickness was measured by using the "scratch" method. The scratches were achieved with a plastic cone tip and were always imaged perpendicular to the fast scan axis. Profilometric section analysis of a scratched film was used to determine the thickness of the film over the scanned area. The film thickness is the minimal z distance between the bare substrate and the surface of the film which covers the whole substrate. The mean thickness of the scratched film was determined by measuring the thickness on at least six areas. The film roughness is the RMS given by the AFM software on 3 $\times$ 3 $\mu m^2$ images. Data evaluations were performed with the NanoScope software version 5.31r1 (Digital Instruments, Veeco).

**Colorimetric enzymatic activity of cross-linked enzyme/polyphenol film**

A multidetector spectroscope UV (Xenius XC, SAFAS, Monaco) equipped with a microplate reader was used to monitor the catalytic activity of the enzyme within the film using o-dianisidine assay. The films were disposed in a 24 wells plate with 1 mL of a solution containing glucose (1 mg/mL), horseradish peroxidase (HRP) (1 mg/mL) and o-dianisidine ($10^{-3}$ M) prepared in 150 mmol/L $NaNO_3$-10 mmol/L Tris buffer at pH = 8.0. The enzyme's activity was monitored by using a second enzyme, HRP, which will use the $H_2O_2$ produced during the reaction of the immobilized enzyme with glucose and will react with o-dianisidine (colourless) to obtain oxidized o-dianisidine, which is brown. The reaction was followed at 440 nm.

**Electrochemical enzymatic activity of cross-linked enzyme/polyphenol film**

All measurements were carried out on a CHI 660B electrochemical workstation (CH Instruments, USA). The same electrode set-up used during the electrodeposition, was used for the electrochemical performance study. The gold electrode was used as the working electrode. Cyclic Voltammetry and Chronoamperometric measurements were carried out after injecting 600 μL of solutions of different concentration of glucose at 1 mL/min in the absence and in the presence of a mediator (0.5 mmol/L of ferrocene methanol, FC). The chronoamperometric measurements of glucose were performed in Ar-saturated solution at constant potential of 0.25 V (vs Ag/AgCl). A surface area of 0.8 $cm^2$, corresponding to the exposed area of the gold QCM sensor, was used for current density calculations as explained in Singh, K.; McArdle, T.; Sullivan, P. R.; Blanford, C. F. "Sources of Activity Loss in the Fuel Cell Enzyme Bilirubin Oxidase", Energy Environ. Sci., 2013, 6, 2460.

The Michaelis-Menten constant ( $K_m^{app}$ ) was determined to evaluate the biological activity of the immobilized enzyme and is estimated using the following derived equation from Lineweaver-Burk equation as disclosed in Kamin, R. A.; Wilson, G. S. "Rotating Ring-Disk Enzyme Electrode for Biocatalysis Kinetic Studies and Characterization of the Immobilized Enzyme Layer", Anal. Chem., 1980, 52, 1198-1205:

$$\frac{1}{i_{ss}} = \left(\frac{K_m^{app}}{i_{max}}\right)\left(\frac{1}{C}\right) + \left(\frac{1}{i_{max}}\right)$$

where $i_{ss}$ is the steady-state current after the addition of substrate, $i_{max}$ is the maximum current measured under saturated substrate condition, and C is the bulk concentration of the substrate. A low $K_m^{app}$ indicates a high enzymatic activity of the immobilized enzyme.

**Enzyme leaking**

Chronoamperometric measurements of glucose were performed in Ar-saturated solution at constant potential of 0.25 V (vs Ag/AgCl) after injection of 600 μL of different concentration of glucose in the presence of 0.5 mmol/L of methanol ferrocene as disclosed in the electrochemical enzymatic activity test method. A surface area of 0.8 $cm^2$, corresponding to the exposed area of the gold QCM sensor, was used for current density calculations. To prove the covalent immobilization of GOX, three sequential washes (for 5 min) by injection of 0.01% of detergent Tween® 20, prepared in phosphate buffer, were performed during the chronoamperometric test. These washes are expected to remove any physically bound GOX.

**EXAMPLES**

**Example 1: Synthesis of polyphenol of formula (III)**

Polyphenol of formula (III) was prepared in two steps from dopamine (CAS 62-31-7, Sigma) and 3,6,9-trioxaundecandioic acid (TUDA, CAS 13887-98-4, Iris Biotech) according to the following Scheme 1:

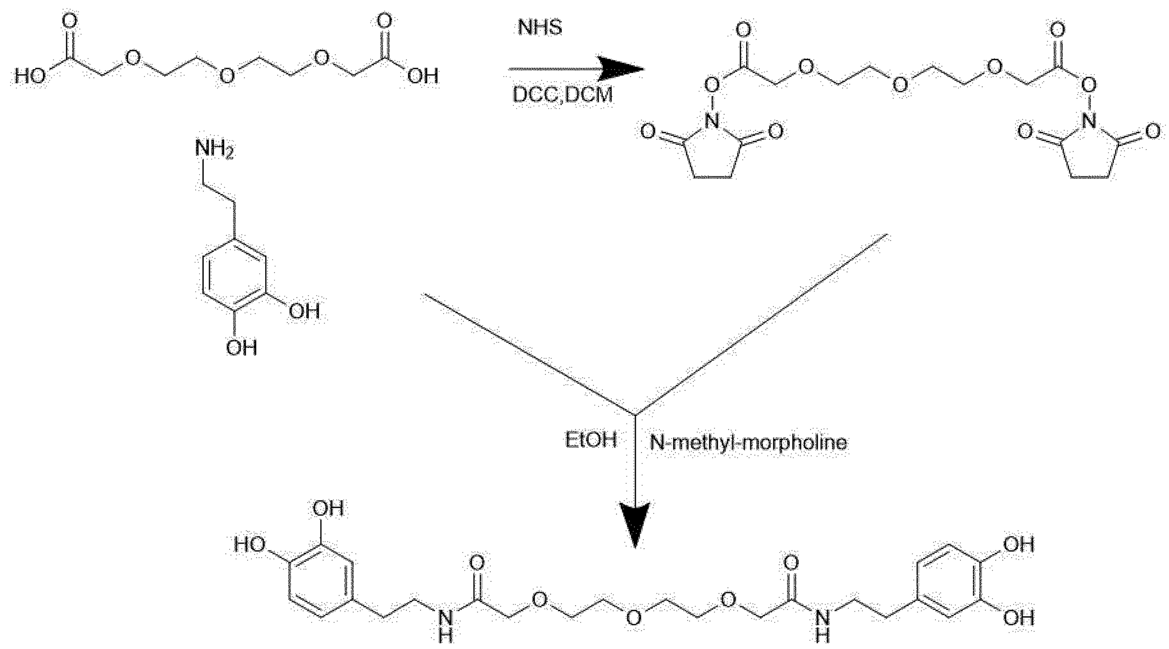


(III)

***Preparation of N,N-succinimide trioxaundecanediamide***

3,6,9-trioxaundecandioic acid (TUDA, 0.99 g, 4.46 mmol, 1.0 eq.) was mixed with 5.31 g of molecular sieves in $CH_2Cl_2$ (20 mL). DCC (3.27 g, 16.19 mmol, 3.6 eq.) and NHS (1.83 g, 15.91 mmol, 3.6 eq.) were added and the mixture was stirred overnight. The solution was filtered over celite. The volume of the solvent was reduced under vacuum. Cold $Et_2O$ was then added to induce the precipitation of the product and the mixture was stored in a cold medium overnight. After filtration, the precipitate was purified with flash chromatography (eluent DCM) affording of solid white product (0.28 g, 15 %). $^1H$ NMR (MeOD-$d^4$, 400 MHz) $\delta$2.83 (s, 8H) 3.68 (m, 4H) 3.79 (m, 4H) 3.78 (m, 4H) 4.52 (s, 4H).

***Preparation of polyphenol of formula (III)***

N,N-succinimide trioxaundecanediamide (0.28 g, 0.67 mmol, 1 eq.) was dissolved in 5 ml of chloroform. Then dopamine hydrochloride (0.25 g, 1.34 mmol, 2 eq.), dissolved in 5 ml of EtOH, was added followed by N-methylmorpholine (0.40 ml, 3.35 mmol, 5 eq.) and the solution was stirred for 48 h at RT. The solvent was evaporated, and the residual solid was purified by flash chromatography eluting with $CH_2Cl_2$/MeOH (1:0 to 97:3) affording 0.25 g of the desired biscatechol of formula (III) (492.52 g/mol, 70 %).

$^1H$ NMR ($D_2O$, 400MHz) $\delta$2.7 (t, J=7.2 Hz, 4H), 3.4 (t, J=7.1 Hz, 4H), 3.52 (s, 8H), 3.96 (s, 4H), 6.68 (dd, *J* = 2 Hz, 8Hz, 2H), 6.77 (d, *J* = 1.9 Hz, 2H), 6.82 (d, *J* = 7.9 Hz, 2H).

***Electrochemical characterization polyphenol of formula (III)***

The electrochemical response of a solution of polyphenol of formula (III) and a mixture of polyphenol of formula (III)/methanol ferrocene was determined by cyclic voltammetry (CV) in phosphate buffer solution (**Figure 2**). In both cases, a pair of redox peaks was observed which corresponds to the transformation of the polyphenol of formula (III) into the corresponding bis-quinone and *vice versa.* In the case of polyphenol of formula (III), the oxidation and reduction peaks are observed at 0.30 V and 0.10 V (*vs.* Ag/AgCl), respectively (**Figure 2a**). This is in good accordance with the literature.

In the presence of methanol ferrocene, the redox peaks are slightly shifted at 0.35 and 0.02 V with higher values of intensity measured, in particular, for the oxidation peak (**Figure 2b**). In both cases, the intensity of oxidation decreases dramatically with the number of cycles. EC-QCM was used to monitor *in situ* the evolution of the normalized frequency shift during the applied CV. An increase of the normalized frequency shift, related to a mass deposition, is observed which might originate from an electro-cross-linking of polyphenol of formula (III), probably through aryloxy radical formation (**Figure 2c**). A higher increase of the normalized frequency shift is observed for the mixture of polyphenol of formula (III)/methanol ferrocene in comparison to the solution of polyphenol of formula (III), reaching a plateau after the

rinsing step at 3800 Hz and 760 Hz, respectively (**Figure 2c**). This shows that ferrocene methanol act as a mediator favoring the oxidation of the polyphenol into the corresponding bis-quinone which can further cross-link to deposit a film. The presence of a morphogen, such as ferrocene methanol, is thus essential for the obtention of the solid composition of the invention.

**Example 2: Synthesis of Rhodamine labeled GOX**

100 mg of GOX was dissolved in 80 ml of a solution of $Na_2CO_3$ (0.1 M, pH 8.5) and stirred at 4°C for 1 h. 340 μl of rhodamine B isocyanate solution (1.5 mg in 0.7 ml of DMSO) was added to the GOX solution and remained stirred at 4°C for 4 h. Rhodamine labeled GOX ($GOX^{Rho}$) was purified by first dialyzing it overnight in a solution of 0.25 M of NaCl and then in pure water for several days.

**Example 3: Preparation of the liquid composition of the invention**

A liquid composition comprising GOX as the protein, the polyphenol of formula (III) and ferrocene methanol as the morphogen was prepared. Knowing that GOX have 15 accessible lysine residues for chemical modification, the [phenol]/[amine] ratio was set to 0.13 to favor the cross-linking of GOX with polyphenol compared to polyphenol self-cross-linking. The liquid composition comprised 1 mg/mL of GOX (6.25 mmol/L), 3 mg/mL polyphenol of formula (III) (6.1 mmol/L) and 0.5 mmol/L of methanol ferrocene in phosphate buffer at pH 7.4. Nitrogen was flushed in the solution to prevent oxidation of the polyphenol due to dissolved oxygen.

**Example 4: Preparation of the solid composition of the invention**

A solid composition comprising the cross-linked product of GOX and the polyphenol of formula (III) was obtained by applying an electric stimulus to the liquid composition of example 3 in the electrochemical cell described above (EC-QCM). Once the QCM signal in contact with the buffer solution was stable, a mixture of the liquid composition of Example 3 was injected in the electrochemical cell (600 μL) at a flow rate of 600 μL/min with a peristaltic pump. After stabilization of the signal, a cyclic voltammetry between 0 and 0.7 V (vs Ag/AgCl, scan rate 0.05 V/s) was applied to trigger the polyphenol oxidation into the corresponding bis-quinone and start the self-construction of the film. A rinsing step was performed by injection in the flux of an aqueous solution of phosphate buffer at the end of the self-construction. The gold working electrode was then un-mounted from the EC-QCM cell and stored into the buffer solution for further characterizations or kept in the cell for electrochemical characterization of the enzymatic activity. The solid composition obtained is referred to GOX/polyphenol film.

**Example 5: Characterization of the solid composition obtained in example 4**

**Figure 3** shows a typical EC-QCM signal relative to the buildup of GOX/polyphenol film obtained in the presence of ferrocene methanol (FC). The injection of polyphenol/GOX/FC mixture induces a small increase of the normalized frequency due to GOX electrostatic adsorption and polyphenol coordination bonding with gold. The application of CV between 0 and 0.7 V (scan rate of 0.05 V/s) results in a fast increase of the normalized frequency followed by a slowdown reaching 610 Hz after 45 min, after the rinsing step. The oxidation currents decrease dramatically with the number of cycles with a superimposition of the last five cycles. The oxido-reduction signal is dominated by FC signal. The oxidation peak is observed at 0.25 V and the reduction peak at 0.11 V shifted to 0.17 V at the end of the CV application. In comparison to polyphenol/FC mixture (Figure 1c), the kinetic of self-construction of polyphenol/GOX/FC mixture is dramatically slower and the electrochemical signals are clearly different. This indicates that different buildup mechanisms are involved in the two cases.

AFM measurements were performed in contact mode and liquid state to characterize the topography and the thickness of the self-constructed films. **Figure 4** shows a homogenous morphology of the different films regardless the deposition time. The films cover uniformly the whole substrate with a thickness varying from of 55 till 100 nm when the film is built for 15 to 60 min, respectively (**Figure 5**). The film thickness increases as a function of the current time application, whereas the film roughness remains constant at about 30 nm until 45 min of self-construction with a little increase at 50 nm for 60 min of buildup.

**Example 6: Biosensing properties of the solid composition obtained in example 4**

The enzymatic activity of GOX/polyphenol film of example 4 was first investigated using the colorimetric enzymatic activity test method described above for different electrodeposition times going from 15 to 60 min. The enzymatic analysis of the different films showed the best response for the self-constructed film at 30 min **(Figure 9a**).

To demonstrate the effective biofunctionalization of the electrode by GOX/polyphenol film of example 4, the electrochemical biosensing capabilities of the immobilized enzyme were determined using standard enzyme-catalyzed glucose oxidation in the presence of ferrocene methanol (FC) as described in the electrochemical enzymatic activity test method above. FC mediator was used to enhance the electron transfer rate between GOX and the electrode and no enzymatic activity was detected in the absence of FC, presumably because the enzymatic active site was inaccessible to direct electron transfer from the electrode. **Figure 6a** displays the sensing mechanism based on the following equations:

$$FC \rightarrow FC^{+} + e^{-} \text{ (at electrode)}$$

$$GOX_{ox} + \beta\text{-D-Glucose} \rightarrow GOX_{red} + \text{D-glucono-}\delta\text{-lactone}$$

$$GOX_{red} + 2\ FC^{+} \rightarrow GOX_{ox} + 2\ H^{+} + 2\ FC$$

In typical electrochemistry test, the reduced form of the mediator, FC, is oxidized into $FC^+$ by the application of an appropriate potential. The introduction of glucose triggers an increase of the anodic current caused by the regeneration of FC through the catalytic cycle depicted in **Figure 7a.** This increase of anodic current contains information relating to the quantity of glucose. GOX/polyphenol films were built for 30 min on gold coated QCM crystal as described in example 4 and further electrochemically characterized in EC-QCM cell by injection of glucose solutions. A phosphate buffer solution (10 mmol/L) at pH 7.4 was chosen as the optimal electrolyte to obtain maximum sensitivity of the biosensor for the measurements. **Figure 6b** shows the different cyclic voltammograms of GOX/polyphenol film in the absence and the presence of 0.5 mmol/L FC at different concentrations of glucose, performed in Ar-saturated environment. In the presence of FC, the oxidation and reduction peaks are observed at 0.27 and 0.16 V, respectively related to the redox behaviors of FC. No redox peaks were observed in pure phosphate buffer or in the presence of 10 mmol/L glucose without mediator. In the presence of FC, the addition of an increasing concentration of glucose leads to a significant increase in the oxidation current and a decrease in the reduction current of the redox couple of FC mediator demonstrating a good bioelectrochemical catalytic activity of GOX/polyphenol film toward glucose oxidation. The potential of 0.25 V was selected as the optimal applied potential for further investigations. **Figure 7a** shows the amperometric response of GOX/polyphenol film. Each fluid replacement led to an electrical current overshoot followed by a period of stabilization at a steady state value. The overshoot happened in the transient period due to artifact noises or a local rise of glucose concentration around the electrode. The steady state value increases with the solution's glucose concentrations. The functionality of GOX and the feasibility of the method for biosensing are confirmed by the increase in current upon addition of successive aliquots of glucose. The calibration curve shows a linear range which extends from 1 to 12.5 mmol/L ($R^2$ = 0.993) on glucose concentration which deviates from linearity at higher concentration representing a typical characteristic of Michaelis-Menten kinetics **(Figure 7b**). This system is thus able to distinguish healthy subjects (3.8 - 6.5 mmol/L) from hyperglycemic subjects (8.3 - 16.6 mmol/L). The average sensitivity, calculated from the slope of the calibration curve, is of 0.66 $\mu$A/(mmol/L).$cm^2$ with a detection capacity of the system at 0.6 mmol/L (Limit of detection, LOD at a signal to noise ratio 3).

The Michaelis-Menten constant ( $K_m^{app}$ ) was determined according to the Electrochemical enzymatic activity test method described above. The $K_m^{app}$ of self-constructed GOX/polyphenol film of example 4 is about 6.3 mM which is lower than the reported 10.36 mM obtained for GOX/polyaniline,[50] 19 mM for GOX/ZnO nanotubes[51] and 21.4 mM GOX/$CaCO_3$[52] biosensors. The above result further indicates that the electrodeposited films exhibit a high affinity to glucose with $I_{max}$ = 25 $\mu$A/mM.

**Example 7: Biosensing selectivity of the solid composition obtained in example 4**

The selectivity of GOX/polyphenol functionalized biosensor was evaluated using common blood interfering substances, such as salicylic acid (SA, 0.75 mM), acetaminophen (AP, 0.35 mM), uric acid (UA, 0.5 mM)) and ascorbic acid (AA, 0.15 mM), which could have a contribution on the amperometric signal because of their low redox potentials. Thus, the maximum common concentration of these molecules in blood (Medscape) was added to the buffer solution both in the absence and the presence of 5 mM glucose to measure the current response at 0.25 V. There are not significant differences in the biosensor response due to the presence of these interfering species, suggesting an excellent anti-interference ability of the biosensor **(Figure 8**).

**Example 8: Determination of enzyme leaking**

The enzymatic activity of GOX/polyphenol film of example 4 was first investigated using the colorimetric enzymatic activity test for different electrodeposition times going from 15 to 60 min. The enzymatic analysis of the different films showed the best response for the self-constructed film at 30 min with no leaking of the GOX from the matrix due to the covalent cross-linking with the polyphenol in the film **(Figure 9a)**.

Enzyme leaking was first assessed by conducting the colorimetric enzymatic activity test (absorbance of the supernatant as a function of time at 440 nm) on the GOX/polyphenol film of example 4 obtained after 30 min of self-construction. The film was removed from the medium after 30 min of enzymatic reaction. The absorbance remained constant after removal of the film from the medium (**Figure 9b**) thus indicating that there was no leaking of GOX from the matrix due to the covalent cross-linking with the polyphenol in the film (no GOX in its free form in the medium).

This conclusion is further confirmed by the enzyme leaking test defined above, since the current densities measured before and after 3 washes with 0.01% of detergent Tween® 20 are similar **(Figure 10)**.

**Example 9: Functionalization of interdigitated array of electrodes**

Since the electro-cross-linking of protein using polyphenol is localized near the electrode, it can be used to functionalize microelectrodes. A solid composition comprising the cross-linked product of rhodamine-labeled GOX ($GOX^{Rho}$) and the polyphenol of formula (III) was obtained by applying an electric stimulus to an interdigitated array electrode (IDA ref: A-012125, Biologic) **(Figure 11)** immersed in a liquid composition comprising $GOX^{Rho}$ (1 mg/mL), the polyphenol of formula (III) (3 mg/mL) and methanol ferrocene (0.5 mmol/L) in phosphate buffer at pH 7.4. The microelectrode was addressed for 45 min through the application of a cyclic voltammetry (between 0 and 0.7 V vs Ag/AgCl, scan rate 0.05 V/s) with a CHI 660e potentiostat/galvanostat in a 3-electrode cell where the working electrode was one of the IDA electrodes, the counter electrode part of the IDA and the reference electrode a no-leak Ag/AgCl reference electrode. A station SARFUS IMAGING HR (Nanolane, Le Mans) was used in bright field and fluorescence mode to image the IDA electrodes. The microelectrodes were imaged by optical microscopy in bright field and in fluorescence to check the presence of $GOX^{Rho}$ and to verify its spatial localization **(Figure 12)**. $GOX^{Rho}$/polyphenol film exhibits an excellent spatio-selectivity since a high fluorescence is observed only on the addressed microelectrodes.

## Claims

1. A liquid composition comprising:

- a protein;
- a polyphenol; and
- a morphogen

wherein said protein is an enzyme or an antibody,
wherein said morphorgen is a ferrocene, and
wherein the polyphenol corresponds to the following formula (II):

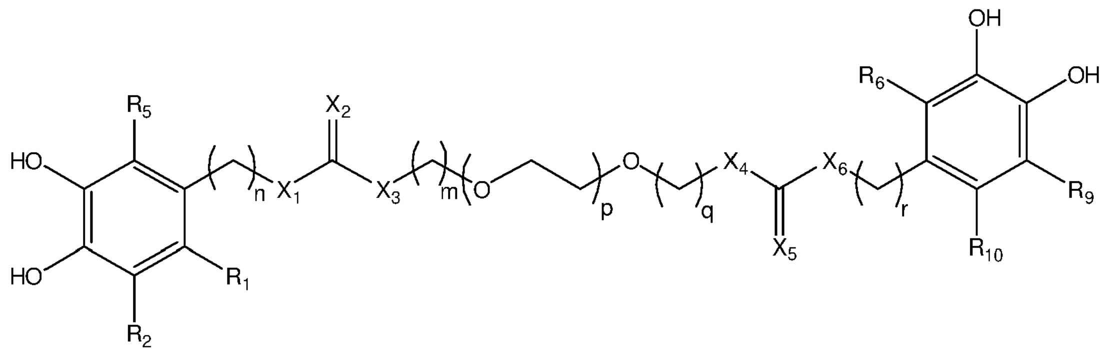

(II)

wherein $R_1$, $R_2$, $R_5$, $R_6$, $R_9$, and $R_{10}$ are each independently selected from H and OH;
$X_1$, $X_3$, $X_4$ and $X_6$ are each independently a bond, O, N or S;
$X_2$ and $X_5$ are each independently O or S;

n, m, p, q are r are integers that are independently equal to 0, 1, 2, 3, 4, 5, 6, 7 or 8, provided that n, m, p, q are r are not all equal to 0.

**2.** The liquid composition of claim 1 wherein the protein is an enzyme, in particular the enzyme is a redox enzyme; more particularly the enzyme is selected from a dehydrogenase, a reductase, an oxidase, an oxygenase, a peroxidase, a catalase, a transhydrogenase, a transferase, a hydrolase, a lyase, an isomerase, a ligase and a urease; even more particularly the enzyme is selected from glucose oxidase (GOX), horseradish peroxidase (HRP), lactate oxidase, alcohol dehydrogenase, aldehyde dehydrogenase, urease, glutamate oxidase, choline oxidase, glucose dehydrogenase, laccase, bilirubin oxidase, ascorbate oxidase, formate dehydrogenase, lactate dehydrogenase, pyruvate dehydrogenase, malate dehydrogenase, p-cresolmethylhydroxylase, methylamine dehydrogenase, succinate dehydrogenase, fumarate reductase, D-fructose dehydrogenase, D-gluconate dehydrogenase, cytochrome c, peroxidase, ferredoxin, plastocyanin, azurin, and azotoflavin; more particularly still the enzyme is GOX.

**3.** The liquid composition of claim 1 or 2, wherein the polyphenol corresponds to the following formula (III):

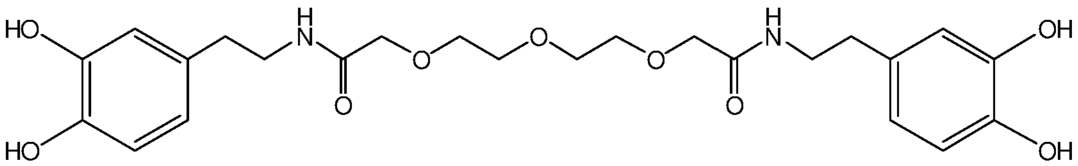

(III) .

**4.** The liquid composition of any one of claims 1 to 3, wherein the morphogen is di(cyclopentadienyl)iron, methanol ferrocene, acetylferrocene, 1,1'-diacetylferrocene, (dimethylaminomethyl)ferrocene, ferrocenecarboxaldehyde, (1-acetoxyethyl)ferrocene, ferrocenoyl azide, α-methylferrocenemethanol, 1-(dimethylamino)ethyl]ferrocene, aminomethylferrocene, 1,1'-di(aminomethyl)ferrocene, aminoethylferrocene, 1,1'-di(aminoethyl)ferrocene, ferrocenecarboxylic acid, 1,1'-ferrocenedicarboxylic acid, (6-Bromo-1-oxohexyl)ferrocene.

**5.** The liquid composition of any one of claims 1 to 4, wherein the morphogen is methanol ferrocene.

**6.** The liquid composition of any one of claims 1-5 which is a buffered solution; in particular a phosphate buffered solution; more particularly a phosphate buffered solution having a pH of 5 to 9, in particular 6 to 8, more particularly 7 to 7.8.

**7.** A solid composition comprising a cross-linked product of a protein and a polyphenol, wherein said protein is an enzyme or an antibody, and

wherein the polyphenol corresponds to the following formula (II):

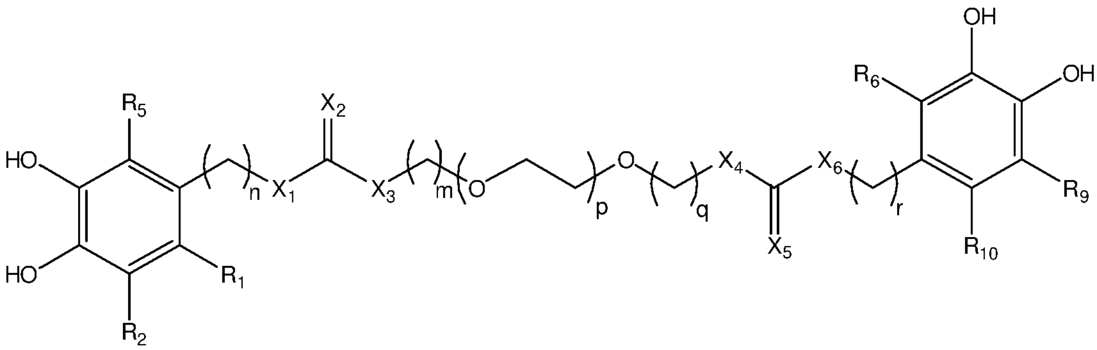

(II)

wherein $R_1$, $R_2$, $R_5$, $R_6$, $R_9$, and $R_{10}$ are each independently selected from H and OH;
$X_1$, $X_3$, $X_4$ and $X_6$ are each independently a bond, O, N or S;
$X_2$ and $X_5$ are each independently O or S;
n, m, p, q are r are integers that are independently equal to 0, 1, 2, 3, 4, 5, 6, 7 or 8, provided that n, m, p, q

are r are not all equal to 0.

**8.** The solid composition of claim 7, wherein the solid composition is in the form of a film.

**9.** The solid composition of claim 8, wherein the film exhibits a thickness of 40 to 150 nm, more particularly 45 to 120 nm, even more particularly 50 to 100 nm.

**10.** A process for the preparation of the solid composition of any one of claims 7-9, wherein the process comprises the steps of:

- contacting an electrochemical probe with the liquid composition as defined in any one of claims 1-6;
- applying an electric stimulus to the electrochemical probe so as to form a solid composition comprising a cross-linked product of a protein and a polyphenol on a surface of the electrochemical probe.

**11.** A biosensor comprising an electrochemical probe wherein a solid composition comprising a cross-linked product of a protein and a polyphenol is bound to a surface thereof, wherein the solid composition is as defined in any one of claims 7 to 9 or obtained by the process of claim 10.

**12.** A biofuel cell comprising:

a positive electrode;
a negative electrode;
an electrolyte; and
an external circuit electrically connecting the positive electrode and the negative electrode;
wherein a solid composition comprising a cross-linked product of a protein and a polyphenol is bound to a surface of the positive electrode and/or the negative electrode, wherein the solid composition is as defined in any one of claims 7 to 9 or obtained by the process of claim 10.

**13.** Use of a biosensor according to claim 11 for the detection of an analyte in a sample.

**Patentansprüche**

**1.** Flüssige Zusammensetzung, umfassend:

- ein Protein;
- ein Polyphenol; und
- ein Morphogen

wobei das Protein ein Enzym oder ein Antikörper ist,
wobei das Morphorgen ein Ferrocen ist, und
wobei das Polyphenol der folgenden Formel (II) entspricht:

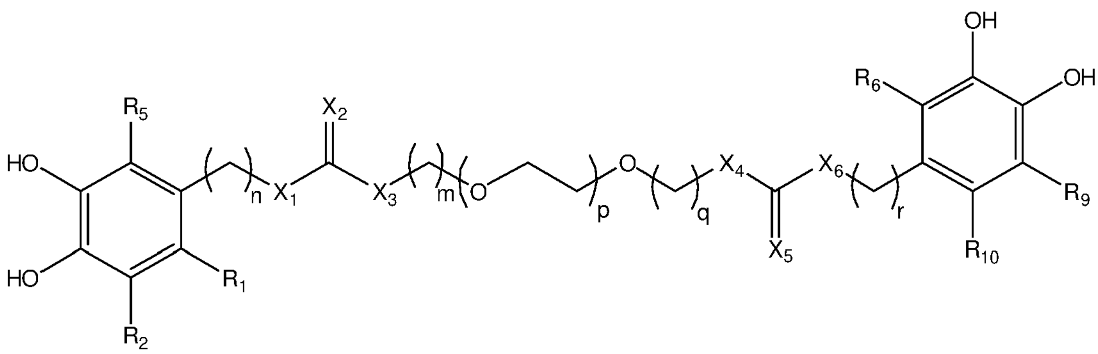

(II)

wobei $R_1$, $R_2$, $R_5$, $R_6$, $R_9$ und $R_{10}$ jeweils unabhängig ausgewählt sind aus H und OH;
$X_1$, $X_3$, $X_4$ und $X_6$ jeweils unabhängig eine Bindung, O, N oder S sind;

$X_2$ und $X_5$ jeweils unabhängig O oder S sind;
n, m, p, q und r ganze Zahlen sind, die unabhängig gleich wie 0, 1, 2, 3, 4, 5, 6, 7 oder 8 sind, vorausgesetzt, dass n, m, p, q und r nicht alle gleich wie 0 sind.

**2.** Flüssige Zusammensetzung nach Anspruch 1, wobei das Protein ein Enzym ist, insbesondere ein Redox-Enzym; insbesondere das Enzym ausgewählt ist aus einer Dehydrogenase, einer Reduktase, einer Oxidase, einer Oxygenase, einer Peroxidase, einer Katalase, einer Transhydrogenase, einer Transferase, einer Hydrolase, einer Lyase, einer isomerase, einer Ligase und einer Urease; insbesondere das Enzym ausgewählt ist aus Glucoseoxidase (**GOX**), Meerrettichperoxidase (**HRP**), Lactatoxidase, Alkoholdehydrogenase, Aldehyddehydrogenase, Urease, Glutamatoxidase, Cholinoxidase, Glucosedehydrogenase, Laccase, Bilirubinoxidase, Ascorbatoxidase, Formiatdehydrogenase, Lactat-Dehydrogenase, Pyruvat-Dehydrogenase, Malat-Dehydrogenase, p-Kresolmethylhydroxylase, Methylamin-Dehydrogenase, Succinat-Dehydrogenase, Fumarat-Reduktase, D-Fructose-Dehydrogenase, D-Gluconat-Dehydrogenase, Cytochrom c, Peroxidase, Ferredoxin, Plastocyanin, Azurin und Azotoflavin; insbesondere das Enzym **GOX** ist.

**3.** Flüssige Zusammensetzung nach Anspruch 1 oder 2, wobei das Polyphenol der folgenden Formel (III) entspricht:

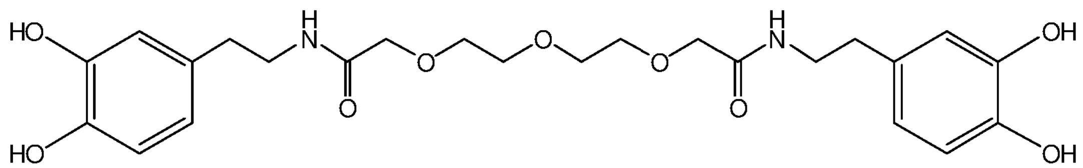

(III)

**4.** Flüssige Zusammensetzung nach einem der Ansprüche 1 bis 3, wobei das Morphogen di(Cyclopentadienyl)eisen, Methanolferrocen, Acetylferrocen, 1,1'-Diacetylferrocen, (Dimethylaminomethyl)ferrocen, Ferrocencarboxaldehyd, (1-Acetoxyethyl)ferrocen, Ferrocenoylazid, α-methylferrocenmethanol, 1-(Dimethylamino)ethyl]ferrocen, Aminomethylferrocen, 1,1'-di(Aminomethyl)ferrocen, Aminoethylferrocen, 1,1'-di(Aminoethyl)ferrocen, Ferrocencarbonsäure, 1,1'-Ferrocendicarbonsäure, (6-Brom-1-oxohexyl)ferrocen ist.

**5.** Flüssige Zusammensetzung nach einem der Ansprüche 1 bis 4, wobei das Morphogen Methanolferrocen ist.

**6.** Flüssige Zusammensetzung nach einem der Ansprüche 1 bis 5, die eine gepufferte Lösung ist, insbesondere eine phosphatgepufferte Lösung, vor allem eine phosphatgepufferte Lösung mit einem pH von 5 bis 9, insbesondere 6 bis 8, vor allem 7 bis 7,8.

**7.** Feste Zusammensetzung, umfassend ein vernetztes Produkt aus einem Protein und einem Polyphenol, wobei das Protein ein Enzym oder ein Antikörper ist, und

wobei das Polyphenol der folgenden Formel (II) entspricht:

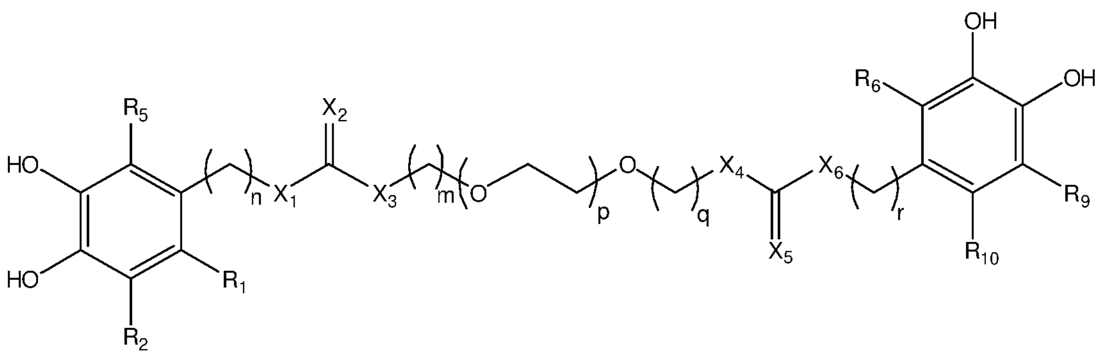

(II)

wobei $R_1$, $R_2$, $R_5$, $R_6$, $R_9$ und $R_{10}$ jeweils unabhängig ausgewählt sind aus H und OH;
$X_1$, $X_3$, $X_4$ und $X_6$ jeweils unabhängig eine Bindung, O, N oder S sind;

$X_2$ und $X_5$ jeweils unabhängig O oder S sind;
n, m, p, q und r ganze Zahlen sind, die unabhängig gleich wie 0, 1, 2, 3, 4, 5, 6, 7 oder 8 sind, vorausgesetzt, dass n, m, p, q und r nicht alle gleich wie 0 sind.

**8.** Feste Zusammensetzung nach Anspruch 7, wobei die feste Zusammensetzung in Form eines Films ist.

**9.** Feste Zusammensetzung nach Anspruch 8, wobei der Film eine Stärke von 40 bis 150 nm, insbesondere 45 bis 120 nm, noch spezifischer 50 bis 100 nm aufweist.

**10.** Verfahren zur Herstellung der festen Zusammensetzung nach einem der Ansprüche 7-9, wobei das Verfahren die folgenden Schritte umfasst:

- Inkontaktbringen einer elektrochemischen Sonde mit der flüssigen Zusammensetzung nach einem der Ansprüche 1-6;
- Anlegen eines elektrischen Reizes an die elektrochemische Sonde, um eine feste Zusammensetzung zu bilden, die ein vernetztes Produkt aus einem Protein und einem Polyphenol auf einer Oberfläche der elektrochemischen Sonde umfasst.

**11.** Biosensor, umfassend eine elektrochemische Sonde, wobei eine feste Zusammensetzung, die ein vernetztes Produkt aus einem Protein und einem Polyphenol umfasst, an eine Oberfläche davon gebunden ist, wobei die feste Zusammensetzung wie definiert in einem der Ansprüche 7 bis 9 ist oder durch das Verfahren nach Anspruch 10 erlangt wird.

**12.** Biobrennstoffzelle, umfassend:

eine positive Elektrode;
eine negative Elektrode;
einen Elektrolyten und
eine externe Schaltung, die die positive Elektrode und die negative Elektrode elektrisch verbindet; wobei eine feste Zusammensetzung, die ein vernetztes Produkt aus einem Protein und einem Polyphenol umfasst, an eine Oberfläche der positiven Elektrode und/oder der negativen Elektrode gebunden ist, wobei die feste Zusammensetzung wie definiert in einem der Ansprüche 7 bis 9 ist oder durch das Verfahren nach Anspruch 10 erlangt wird.

**13.** Verwendung eines Biosensors nach Anspruch 11 für den Nachweis eines Analyten in einer Probe.

**Revendications**

**1.** Composition liquide comprenant :

- une protéine ;
- un polyphénol ; et
- un morphogène

dans laquelle ladite protéine est une enzyme ou un anticorps,
dans lequel ledit morphorgène est un ferrocène, et
dans lequel le polyphénol correspond à la formule (II) suivante :

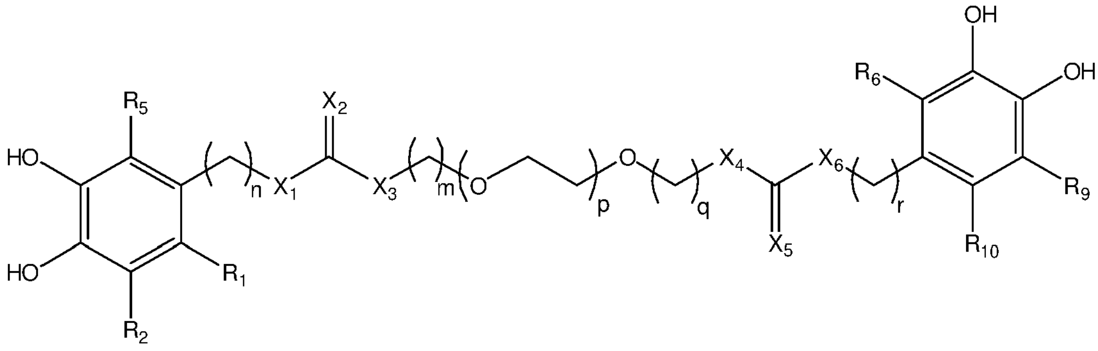

(II)

dans lequel $R_1$, $R_2$, $R_5$, $R_6$, $R_9$, et $R_{10}$ sont chacun sélectionnés indépendamment parmi H and OH ;
$X_1$, $X_3$, $X_4$ et $X_6$ sont chacun indépendamment une liaison, O, N ou S ;
$X_2$ et $X_5$ sont chacun indépendamment O ou S ;
n, m, p, q et r sont des nombres entiers indépendamment égaux à 0, 1, 2, 3, 4, 5, 6, 7 ou 8, à condition que n, m, p, q et r ne soient pas tous égaux à 0.

**2.** Composition liquide selon la revendication 1 dans laquelle la protéine est une enzyme, en particulier l'enzyme est une enzyme redox ; plus particulièrement l'enzyme est choisie parmi une déshydrogénase, une réductase, une oxydase, une oxygénase, une peroxydase, une catalase, une transhydrogénase, une transférase, une hydrolase, une lyase, une isomérase, une ligase et une uréase ; plus particulièrement encore, l'enzyme est choisie parmi la glucose oxydase (**GOX**), la peroxydase de raifort (**HRP**), la lactate oxydase, l'alcool déshydrogénase, l'aldéhyde déshydrogénase, l'uréase, la glutamate oxydase, la choline oxydase, la glucose déshydrogénase, la laccase, la bilirubine oxydase, l'ascorbate oxydase, la formate déshydrogénase, la lactate déshydrogénase, la pyruvate déshydrogénase, la malate déshydrogénase, la p-crésolméthylhydroxylase, la méthylamine déshydrogénase, la succinate déshydrogénase, la fumarate réductase, la D-fructose déshydrogénase, la D-gluconate déshydrogénase, le cytochrome c, la peroxydase, la ferredoxine, la plastocyanine, l'azurine et l'azotoflavine ; plus particulièrement encore, l'enzyme est la **GOX**.

**3.** Composition liquide selon la revendication 1 ou 2, dans laquelle le polyphénol correspond à la formule (III) suivante :

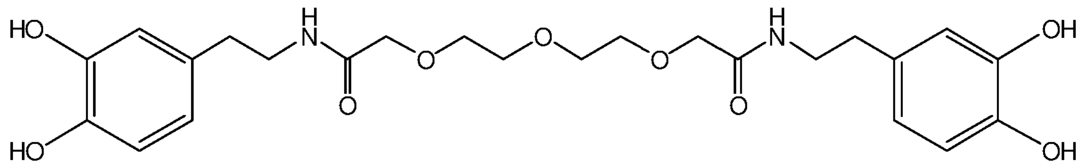

(III)

**4.** Composition liquide selon l'une quelconque des revendications 1 à 3, dans laquelle le morphogène est le di(cyclopentadiényl)fer, le méthanol ferrocène, l'acétylferrocène, le 1,1'-diacétylferrocène, le (diméthylaminométhyl)ferrocène, le ferrocène-carboxaldéhyde, le (1-acétoxyéthyl)ferrocène, l'azoture de ferrocénoyle, le $\alpha$-méthylferrocèneméthanol, le 1-(diméthylamino)éthylferrocène, l'aminométhylferrocène, le 1,1'-di(aminométhyl)ferrocène, l'aminoéthylferrocène, le 1,1'-di(aminoéthyl)ferrocène, l'acide ferrocène-carboxylique, l'acide 1,1'-ferrocènedicarboxylique, le (6- Bromo-1-oxohexyl)ferrocène.

**5.** Composition liquide selon l'une quelconque des revendications 1 à 4, dans laquelle le morphogène est le méthanol ferrocène.

**6.** Composition liquide selon l'une quelconque des revendications 1 à 5 qui est une solution tamponnée ; en particulier une solution tamponnée au phosphate ; plus particulièrement une solution tamponnée au phosphate ayant un pH de 5 à 9, en particulier de 6 à 8, plus particulièrement de 7 à 7,8.

**7.** Composition solide comprenant un produit réticulé d'une protéine et d'un polyphénol, dans laquelle ladite protéine est une enzyme ou un anticorps, et

dans laquelle le polyphénol correspond à la formule (II) suivante :

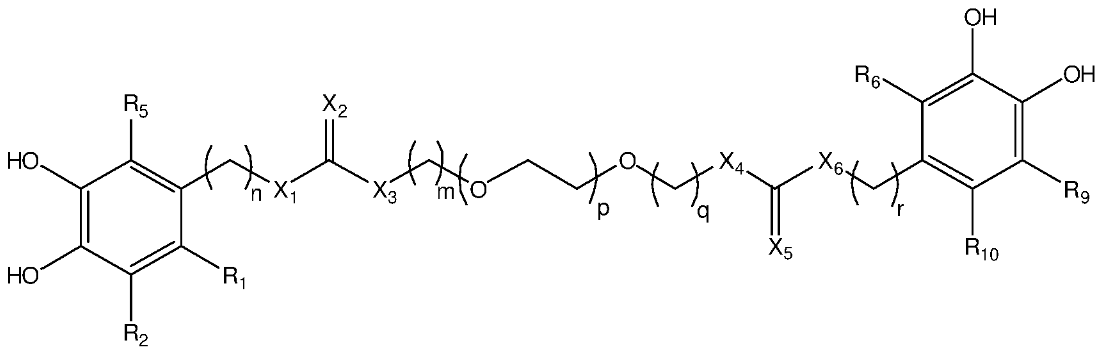

(II)

dans laquelle $R_1$, $R_2$, $R_5$, $R_6$, $R_9$, et $R_{10}$ sont chacun sélectionnés indépendamment parmi H and OH ;
$X_1$, $X_3$, $X_4$ et $X_6$ sont chacun indépendamment une liaison, O, N ou S ;
$X_2$ et $X_5$ sont chacun indépendamment O ou S ;
n, m, p, q et r sont des nombres entiers indépendamment égaux à 0, 1, 2, 3, 4, 5, 6, 7 ou 8, à condition que n, m, p, q et r ne soient pas tous égaux à 0.

**8.** Composition solide selon la revendication 7, dans laquelle la composition solide se présente sous la forme d'un film.

**9.** Composition solide selon la revendication 8, dans laquelle le film présente une épaisseur de 40 à 150 nm, plus particulièrement de 45 à 120 nm, encore plus particulièrement de 50 à 100 nm.

**10.** Procédé de préparation de la composition solide selon l'une quelconque des revendications 7 à 9, dans lequel le procédé comprend les étapes de :

- mise en contact d'une sonde électrochimique avec la composition liquide selon l'une quelconque des revendications 1 à 6 ;
- l'application d'un stimulus électrique à la sonde électrochimique de manière à former une composition solide comprenant un produit réticulé d'une protéine et d'un polyphénol sur une surface de la sonde électrochimique.

**11.** Biocapteur comprenant une sonde électrochimique dans lequel une composition solide comprenant un produit réticulé d'une protéine et d'un polyphénol est lié à une surface de celui-ci, dans lequel la composition solide est telle que définie dans l'une quelconque des revendications 7 à 9 ou obtenue par le procédé selon la revendication 10.

**12.** Pile à combustible biologique comprenant :

une électrode positive ;
une électrode négative ;
un électrolyte ; et
un circuit externe reliant électriquement l'électrode positive et l'électrode négative ; dans laquelle une composition solide comprenant un produit réticulé d'une protéine et d'un polyphénol est lié à une surface de l'électrode positive et/ou de l'électrode négative, dans laquelle la composition solide est telle que définie dans l'une quelconque des revendications 7 à 9 ou obtenue par le procédé selon la revendication 10.

**13.** Utilisation d'un biocapteur selon la revendication 11 pour la détection d'un analyte dans un échantillon.

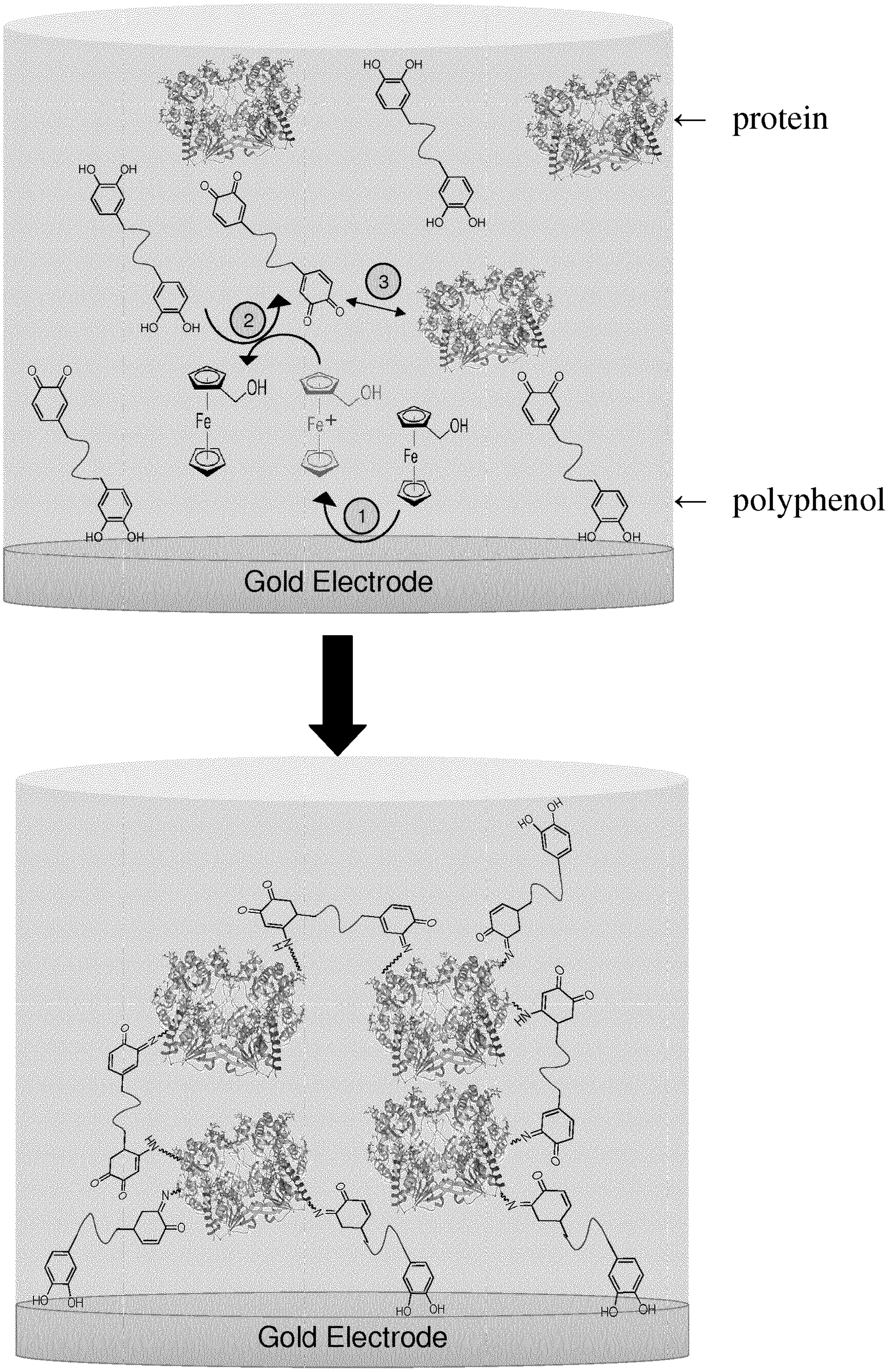


**Figure 1**

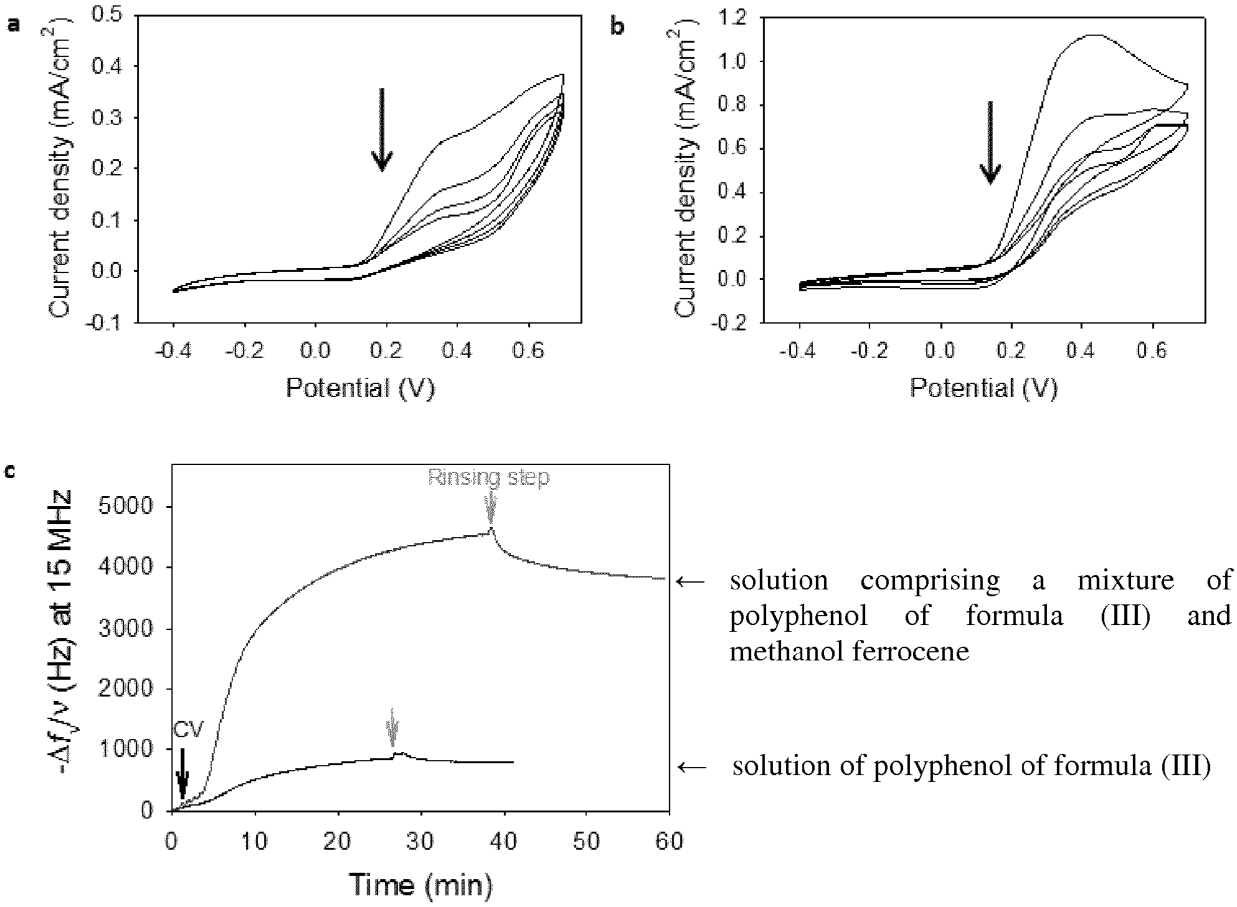
a
b
c
Current density (mA/cm$^2$)
Potential (V)
-Δf/ν (Hz) at 15 MHz
Time (min)
Rinsing step
CV
← solution comprising a mixture of polyphenol of formula (III) and methanol ferrocene
← solution of polyphenol of formula (III)

**Figure 2**

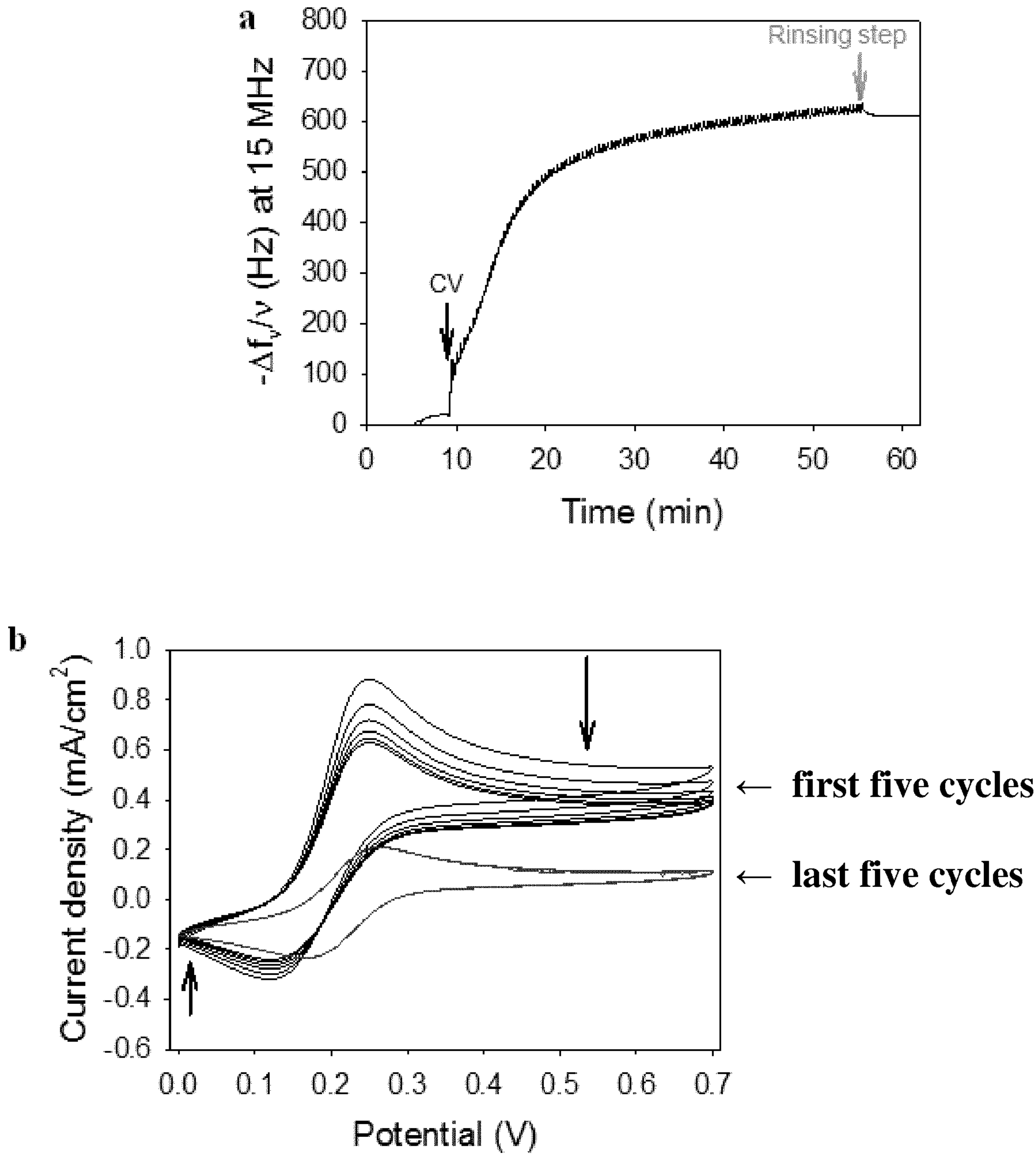
a
Rinsing step
CV
-Δf_ν/ν (Hz) at 15 MHz
Time (min)
b
Current density (mA/cm²)
Potential (V)
← first five cycles
← last five cycles

Figure 3

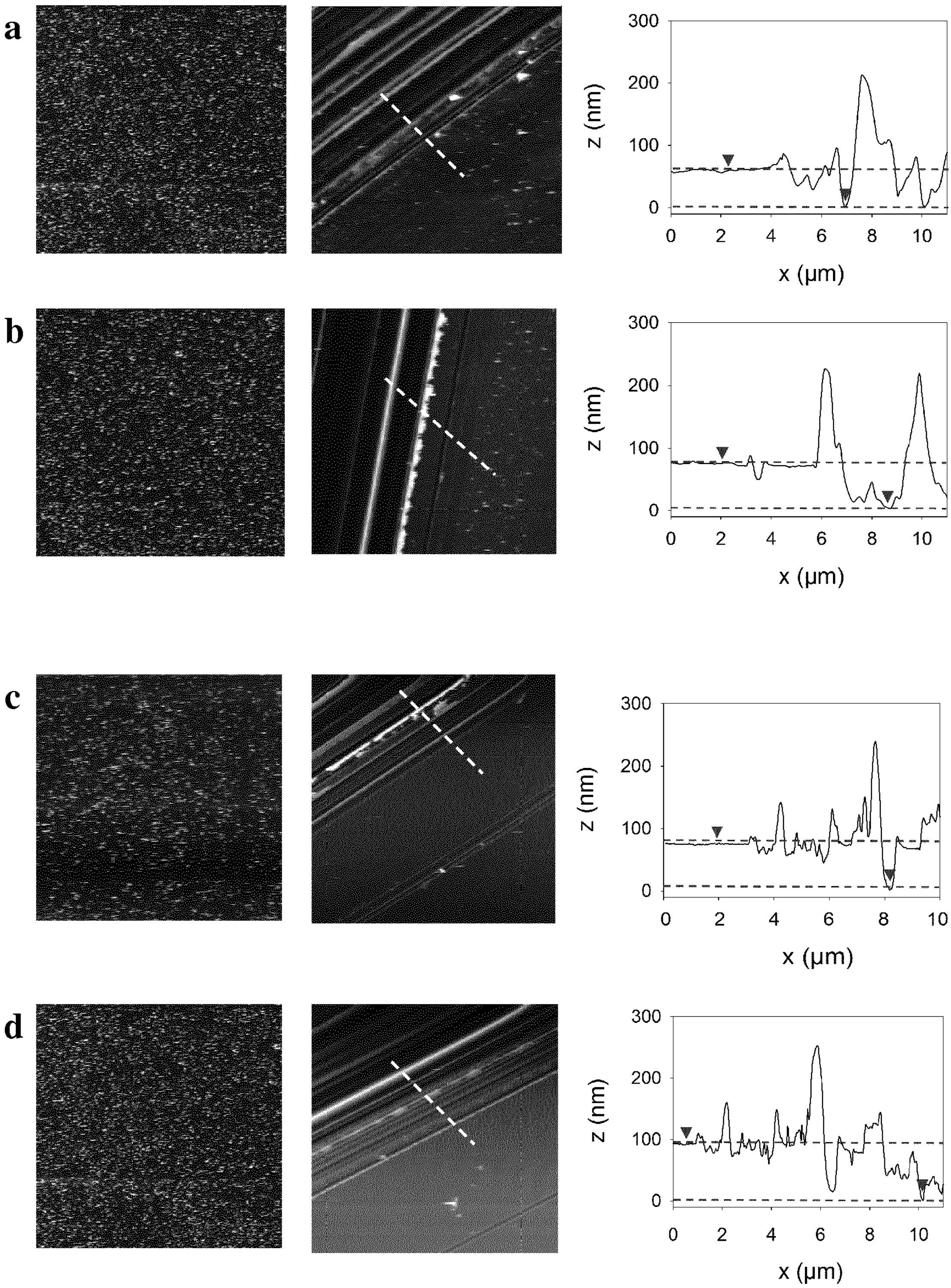


Figure 4

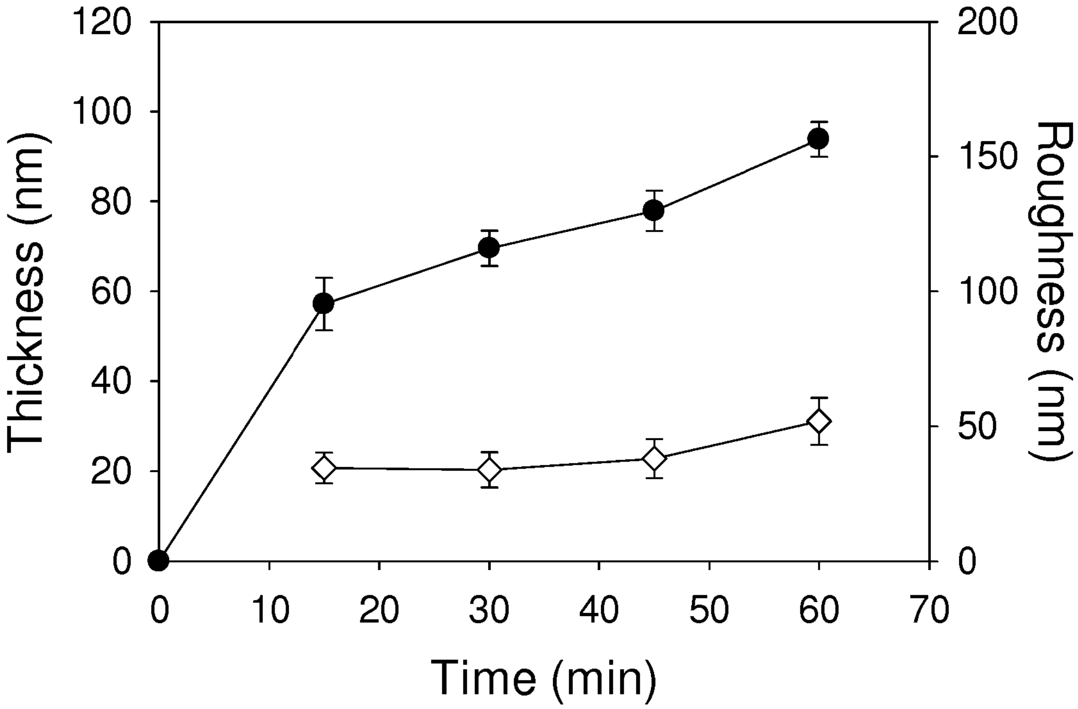
120
100
80
60
40
20
0
Thickness (nm)
200
150
100
50
0
Roughness (nm)
0
10
20
30
40
50
60
70
Time (min)

**Figure 5**

a

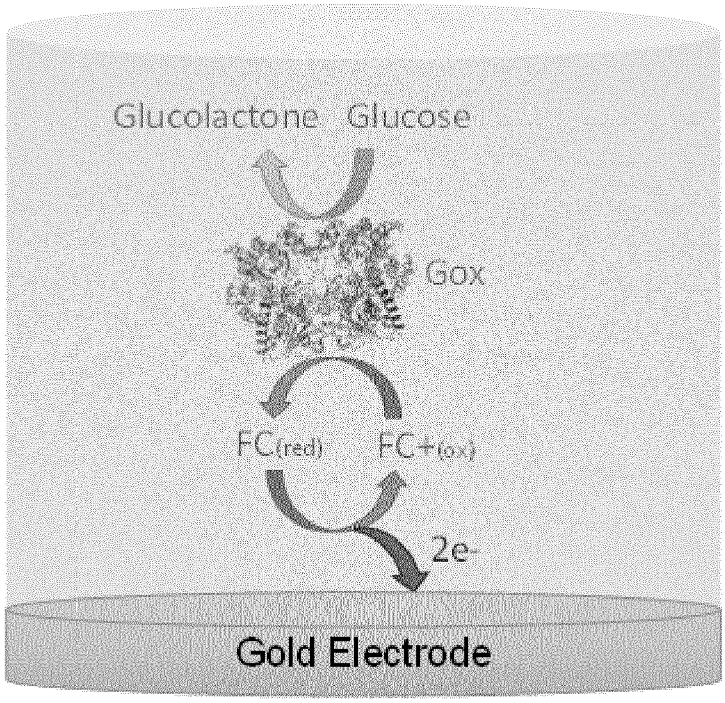
Glucolactone
Glucose
Gox
FC(red)
FC+(ox)
2e-
Gold Electrode

b

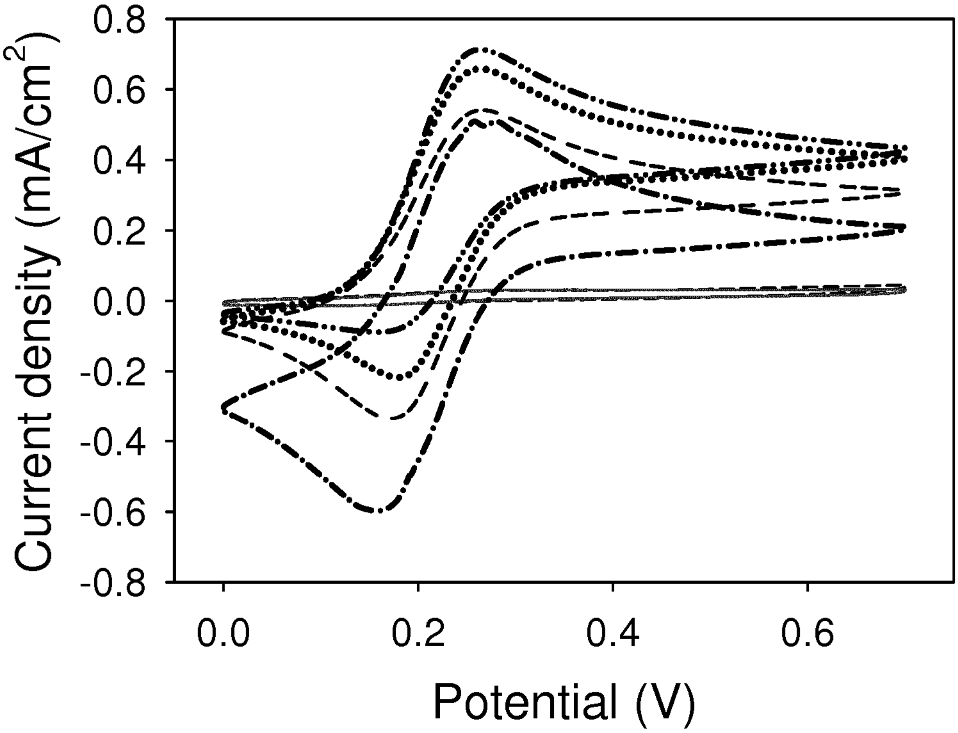
Current density (mA/cm2)
0.8
0.6
0.4
0.2
0.0
-0.2
-0.4
-0.6
-0.8
0.0
0.2
0.4
0.6
Potential (V)

**Figure 6**

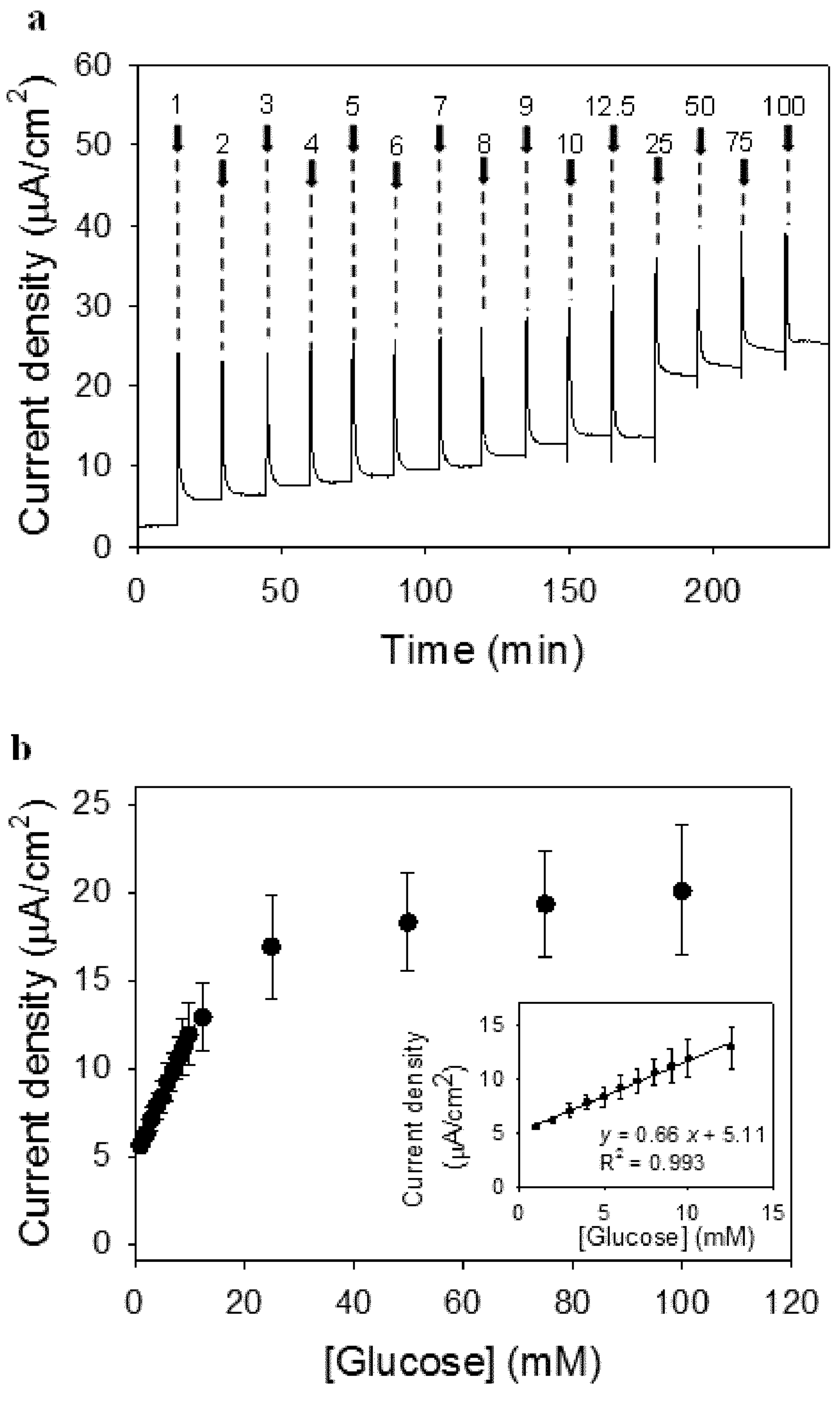
a
Current density (µA/cm2)
Time (min)
1
2
3
4
5
6
7
8
9
10
12.5
25
50
75
100
b
Current density (µA/cm2)
[Glucose] (mM)
y = 0.66 x + 5.11
R2 = 0.993

**Figure 7**

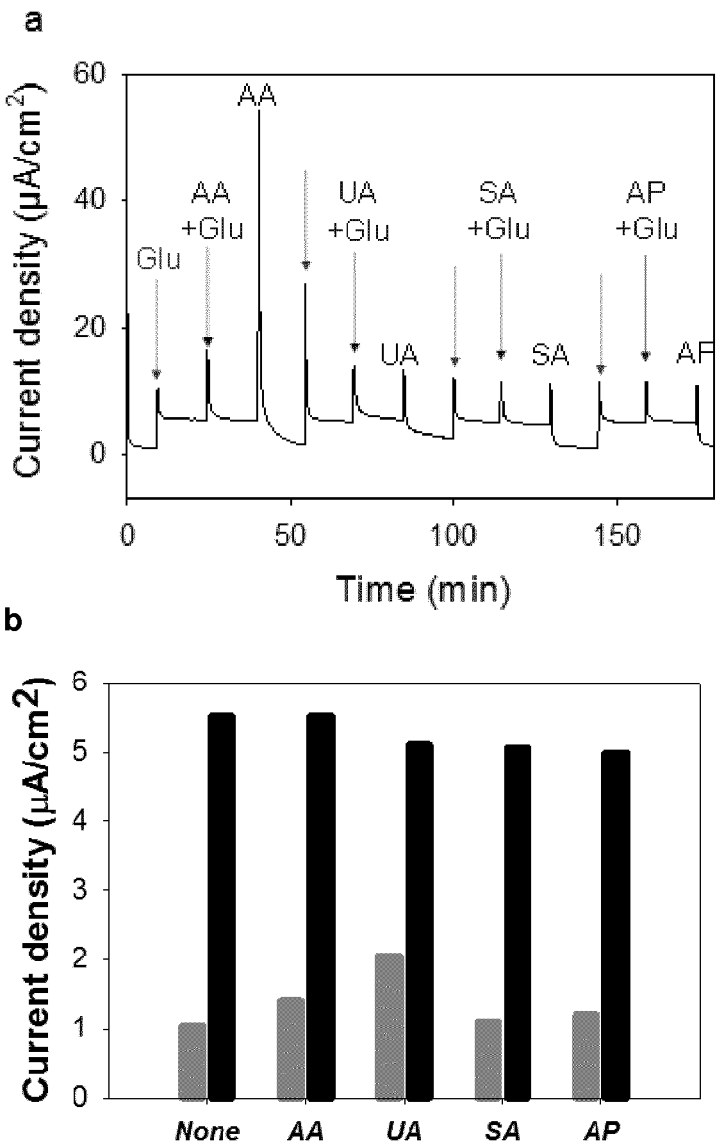
a
Current density (μA/cm2)
Time (min)
Glu
AA +Glu
AA
UA +Glu
UA
SA +Glu
SA
AP +Glu
AP
0
20
40
60
0
50
100
150
b
Current density (μA/cm2)
0
1
2
3
4
5
6
None
AA
UA
SA
AP

**Figure 8**

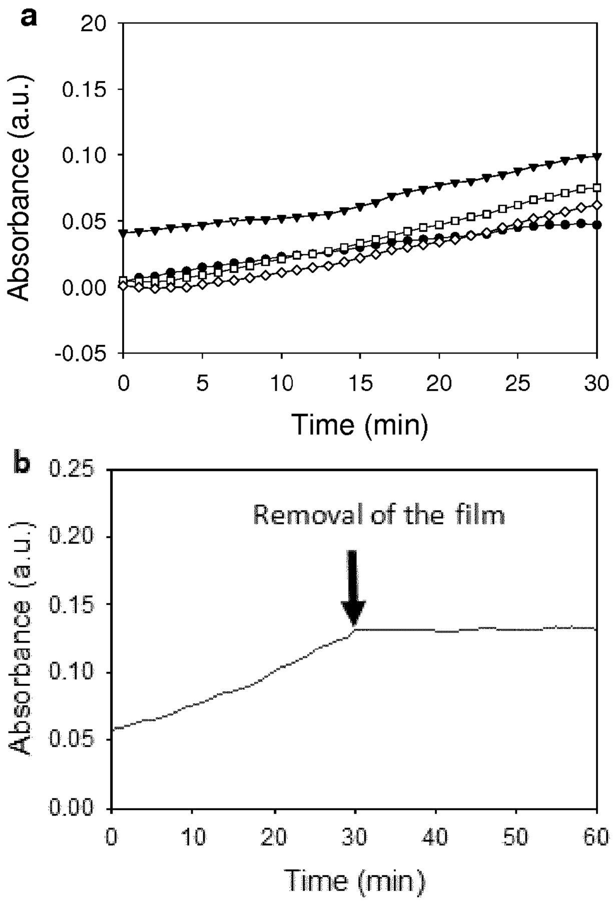


**Figure 9**

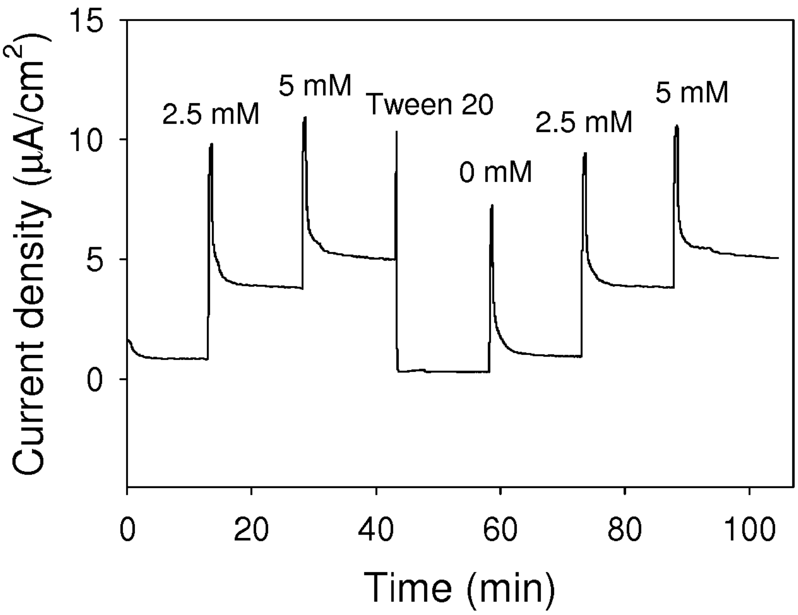


**Figure 10**

a

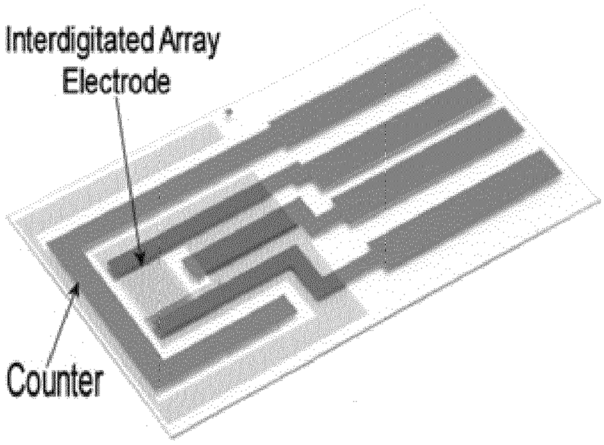
Interdigitated Array
Electrode
Counter

b

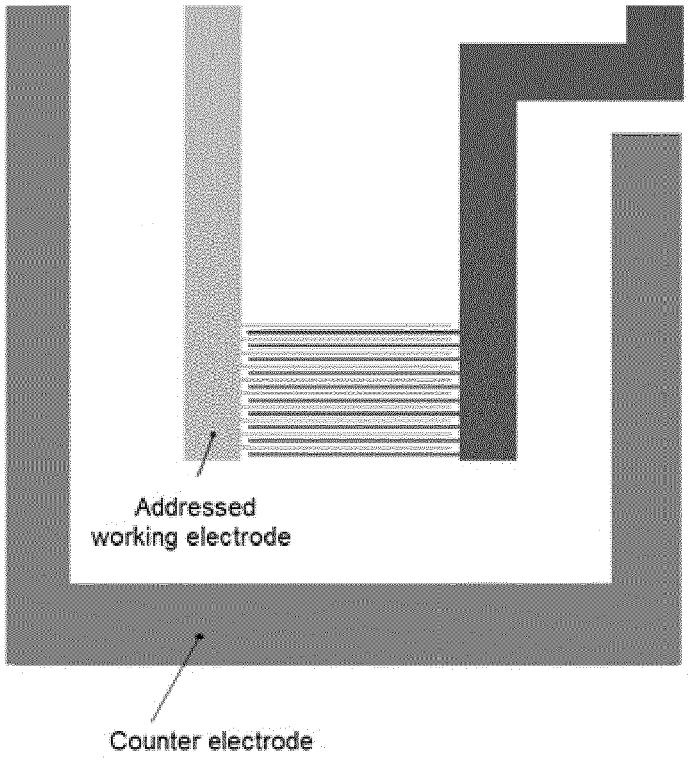
Addressed
working electrode
Counter electrode

**Figure 11**

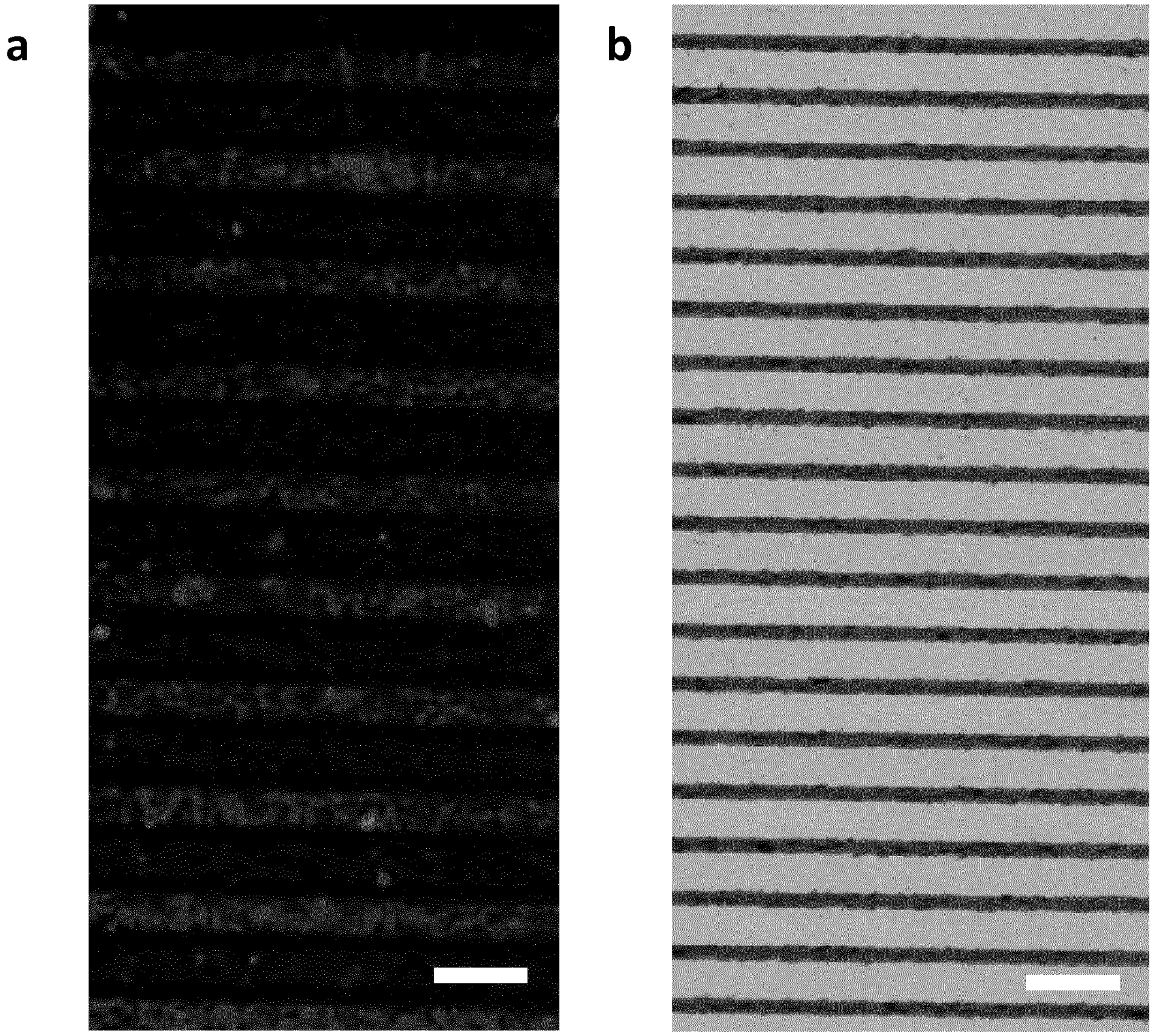

Figure 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2003104119 A


### Non-patent literature cited in the description


- **RYDZEK, G. et al.** Electrochemically Triggered Assembly of Films: A One-Pot Morphogen-Driven Buildup. *Angew. Chemie - Int. Ed.,* 2011, vol. 50, 4374-4377
- **MAERTEN, C. et al.** Electrochemically Triggered Self-Construction of Polymeric Films Based on Mussel-Inspired Chemistry. *Langmuir,* 2015, vol. 31, 13385-13393
- **SIEBERT et al.** *Journal of Agricultural and Food Chemistry,* 1999, vol. 47 (2
- **EDDY et al.** *Biosensors and Bioelectronics,* 1994, vol. 10 (9/10
- **BARTLETT et al.** *Biosensors,* 1987, vol. 3 (6
- **BARTLETT et al.** *Journal of Electroanalytical Chemistry and Interfacial Electrochemistry,* 1993, vol. 362 (1-2
- **ZHANG et al.** *Electrochimica Acta,* 2004, vol. 49 (26
- **PÖLLER et al.** *Electrochimica Acta,* 2013, vol. 110
- **DUMONT et al.** *Biotechnology and Bioengineering,* vol. 49 (5), 1996
- *CHEMICAL ABSTRACTS,* 9001-78-9
- *CHEMICAL ABSTRACTS,* 9001-37-0
- *CHEMICAL ABSTRACTS,* 4264-83-9
- *CHEMICAL ABSTRACTS,* 69-72-7
- *CHEMICAL ABSTRACTS,* 7631-99-4
- *CHEMICAL ABSTRACTS,* 14459-95-1
- *CHEMICAL ABSTRACTS,* 119-90-4
- *CHEMICAL ABSTRACTS,* 50-99-7
- *CHEMICAL ABSTRACTS,* 77-86-1
- *CHEMICAL ABSTRACTS,* 1273-86-5
- *CHEMICAL ABSTRACTS,* 103-90-2
- *CHEMICAL ABSTRACTS,* 69-93-2
- **SINGH, K. ; MCARDLE, T. ; SULLIVAN, P. R. ; BLANFORD, C. F.** Sources of Activity Loss in the Fuel Cell Enzyme Bilirubin Oxidase. *Energy Environ. Sci.,* 2013, vol. 6, 2460
- **KAMIN, R. A. ; WILSON, G. S.** Rotating Ring-Disk Enzyme Electrode for Biocatalysis Kinetic Studies and Characterization of the Immobilized Enzyme Layer. *Anal. Chem.,* 1980, vol. 52, 1198-1205
- *CHEMICAL ABSTRACTS,* 62-31-7
- *CHEMICAL ABSTRACTS,* 13887-98-4